# EUROPEAN PATENT SPECIFICATION

(11) **EP 0 237 045 B1**
(45) Date of publication and mention of the grant of the patent: **20.10.1993**
(21) Application number: 87103488.0
(22) Date of filing: 11.03.1987
(51) Int. Cl.: C12N 15/62, C12P 21/02

(54) **DNA encoding flagellin and vector having the same**
Für Flagellin kodierende DNS und dieses enthaltender Vektor
ADN codant pour la flagelline et vecteur le contenant

(30) Priority: 11.03.1986 JP 54400/86; 19.09.1986 JP 223484/86
(43) Date of publication of application: 16.09.1987
(73) Proprietor: SHIONOGI & CO., LTD., Osaka 541 (JP)
(72) Inventor: Asaka, Junichiro, Sakai-shi Osaka (JP); Fujiwara, Tamio, Amagasaki-shi Hyogo (JP); Fujiwara, Takashi, Nara-shi Nara (JP); Kuwajima, Goro, 5-11, Omori-cho Nishi nomiya-shi Hyogo (JP); Kondo, Eiji, Ikeda-shi Osaka (JP); Shin, Masaru, Kobe-shi Hyogo (JP)
(74) Representative: VOSSIUS & PARTNER

(56) References cited:
- WO-A-87/02385
- JOURNAL OF BIOLOGICAL CHEMISTRY, vol. 260, no. 29, 15th December 1985, pages 15758-15761, The American Society of Biological Chemists Inc.; T.M. JOYS: "The covalent structure of the phase-1 flagellar filament protein of salmonella typhimurium and its comparison with other flagellins"
- THE JOURNAL OF BIOLOGICAL CHEMISTRY, vol. 256, no. 12, 25th June 1983, pages 7395-7401; P.R. GILL et al.: "The nucleotide sequence of the Mr = 28,500 flagellin gene of caulobacter crescentus"
- THE JOURNAL OF BIOLOGICAL CHEMISTRY, vol. 251, no. 3, 10th February 1976, pages 705-711, US; R.J. DELANGE et al.: "Amino acid sequence of flagellin of bacillus subtilis 168"
- JOURNAL OF BACTERIOLOGY, vol. 155, no. 1, July 1983, pages 74-81, American Society for Microbiology; E. SZEKELY et al.: "DNA sequence adjacent to flagellar genes and evolution of flagellar-phase variation"
- JAPANESE JOURNAL OF GENETICS, vol. 60, no. 6, page 589; T. HANAFUSA et al.: "Operator mutations of hag, an E. coli flagellin gene and nucleotide sequences of mutant operator regions"
- JOURNAL OF BACTERIOLOGY, vol. 168, no. 3, December 1986, pages 1479-1483, American Society for Microbiology; G. KUWAJIMA et al.: "Nucleotide sequence of the hag gene encoding flagellin of escherichia coli"

## Description

This invention relates to a DNA having a DNA sequence encoding a flagellin protein lacking central amino acid residues which are unnecessary for excretion in bacteria and an excretion vector carrying said DNA sequence.

The recent development of gene engineering made it possible to insert a gene encoding a certain peptide into a vector and then to introduce the vector into a bacterium such as *Escherichia coli* to get a large amount of the peptide. However, if a peptide expressed in a bacterium is accumulated therein, growth of the bacterium is inhibited by the peptide and production of the peptide is restricted by negative-feedback through excess production of the peptide. In order to recover the peptide, it is necessary to collect and destroy the bacterium, and to purify the peptide from the cell debris which contains a lot of impurities, some of which are pathogenic to human beings, so that it is hard to recover the pure peptide therefrom.

Peptides comprising the cell membrane, secreted peptides and so on are synthesized as pro-peptides having a signal sequence at their N-terminal, which is necessary for their passing through the inner or outer membrane. The pro-peptide, from which the signal sequence is cut off by a peptidase in the membrane when passing through the membrane, becomes a mature peptide showing its original activity and function.

In order to overcome the difficulties in refining the target peptide accumulated in the cells and to improve productivity of the target peptide, it has been attempted to induce the cells to secrete the target peptide out of the cell by utilizing the natural secretion system of the cell. Examples therefore are the secretion of β-lactamase of *Escherichia coli* by a secretion vector carrying the promoter and a DNA encoding the signal sequence of α-amylase of *Bacillus subtilis* (J. Biochem. 95, 87-93 (1984)), the secretion of mouse IFN-β by the same vector system as noted above (Gene 34, 1-8 (1985)) and the secretion of human IFN-α by a secretion vector carrying the promoter and a DNA encoding the signal sequence of alkaline phosphatase of *Escherichia coli* (J. Biochem. 97, 1429-1436 (1985)).

Besides the secretion system as mentioned above, an excretion system is well known by which a peptide synthesized in a cell can leave the cell. For example, flagellin composing the flagella of bacetia is excreted by this excretion system. In the excretion system, differing from the secretion system, a peptide synthesized in a cell having no signal sequence is excreted and presents its function without digestion by a peptidase (J. Bacteriol. 159, 1056-1059 (1984)). The gene encoding flagellin of *E . coli* is called "hag gene", which has already been cloned in pBR322 and phage λ (Abstracts of the 57th annual meeting of Japanese society of genetics, 63 (1985); J. Bacteriol. 130, 736-745 (1977)). Though its partial DNA sequence was analyzed (J. Bacteriol. 155, 74-81 (1983)), the whole DNA sequence has not yet been determined.

The secretion of a peptide depends upon the type of the secretion vector, that is a certain type of vector carrying a certain promoter and a DNA sequence encoding a signal peptide can be used in the secretion of only a few kinds of peptides but not of all kinds of peptides.

The hag gene encoding flagellin of *E. coli* has already been cloned and its sequence has been determined (WO-A-8702385; Szekely et al., J. Bacteriol. 155(1983), 74-81). It has not been suggested at all that the excretion system of flagellin can be utilized for the excretion of a target peptide out of a cell. Moreover, an excretion vector carrying the hag gene has not yet been constructed. The elucidation of the whole sequence of the hag gene made it possible to construct such a vector.

Now, even if a target peptide is secreted into a broth by, utilizing a secretion vector, it is fairly difficult to recover and refine the peptide from the broth. However, if a target peptide is excreted as a peptide fused with flagellin and the flagellin forms flagella thereby, becoming insoluble, it becomes easier to recover the peptide.

Thus, the technical problem underlying the present invention is to provide an improved system for excretion of foreign peptides in transformed bacteria using the excretion system of flagella.

The solution to the above technical problem is achieved by providing the embodiments characterized in the claims.

The whole sequence of DNA coding for flagellin of *E. coli* (hag gene) cloned in phage λ as noted above was determined by the present inventors. The hag gene is inserted into pBR322 and the region of the hag gene responsible for the antigencity of flagella is deleted. Into the resulting space linkers having suitable restriction sites are inserted. When a vector carrying foreign DNA in the linker is introduced into E. coli which are not capable of forming flagella, the E. coli become able of forming flagella and show motility. The flagella comprises flagellin fused with the foreign peptide encoded by the foreign DNA. Namely, the foreign peptide is excreted out of the bacteria. If the foreign peptide fused with the flagellin is a part responsible for the antigencity of the foreign peptide, the transformant does not show motility in a medium containing the antibody against the foreign peptide. Therefore, this system can be utilized for determination of epitopes and preparation of antigens.

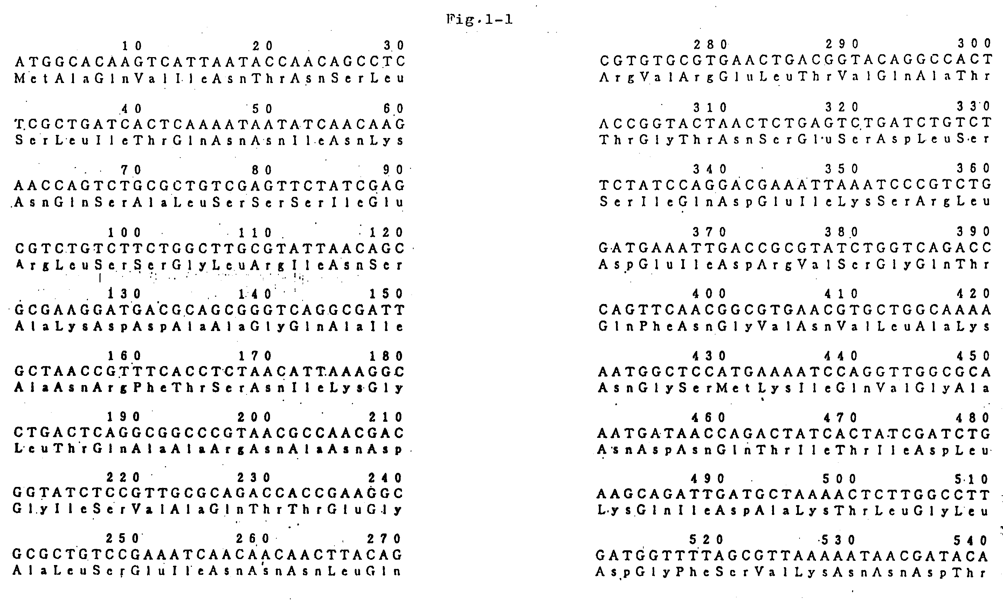

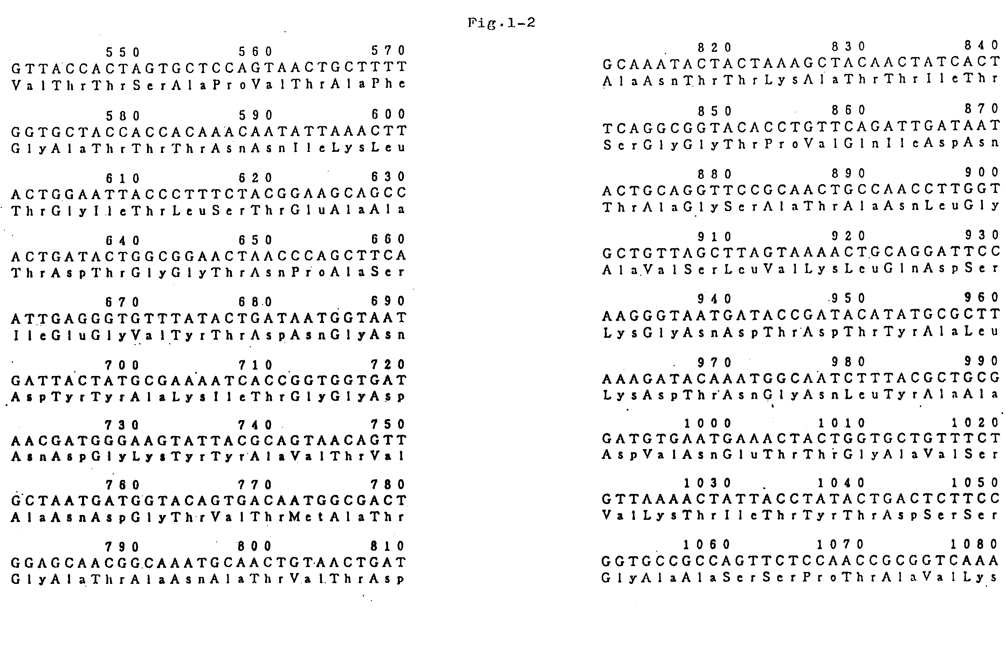

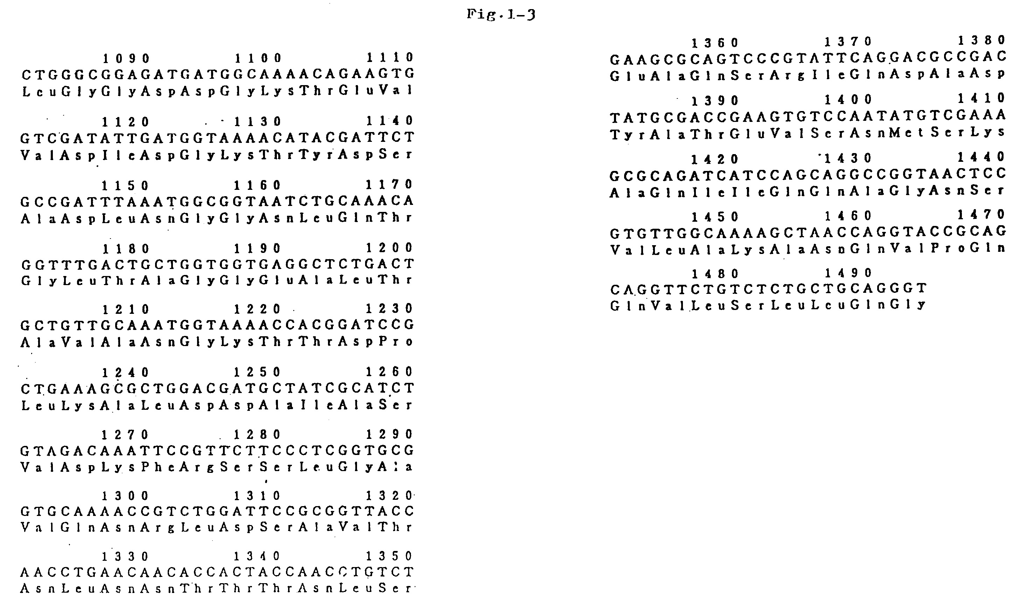

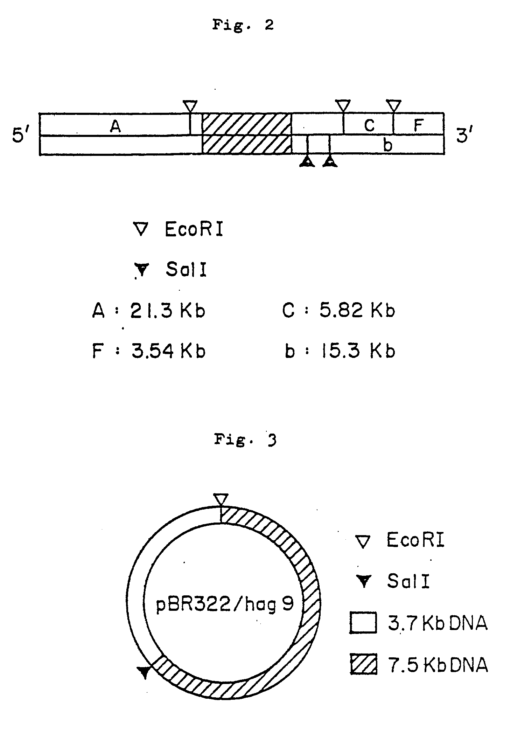

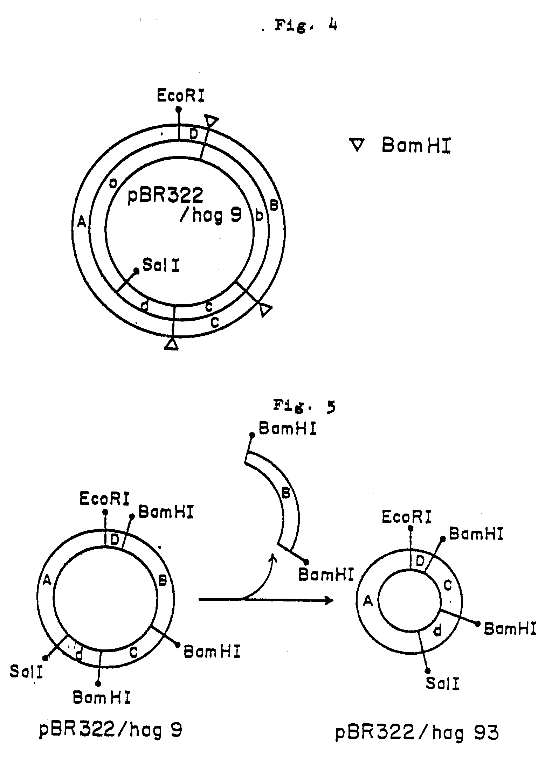

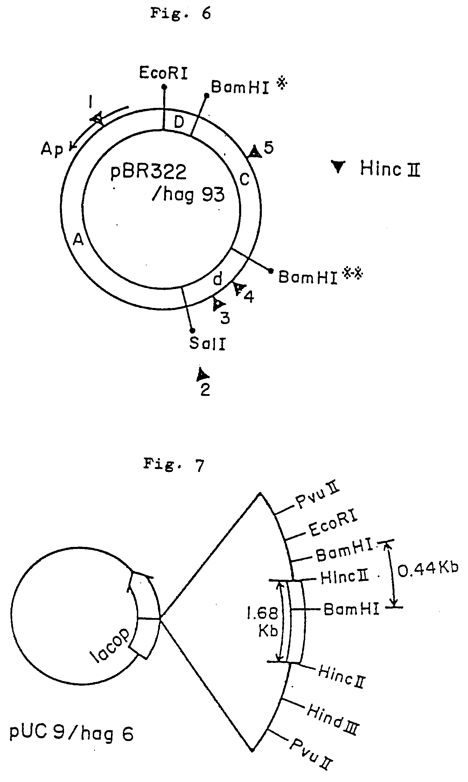

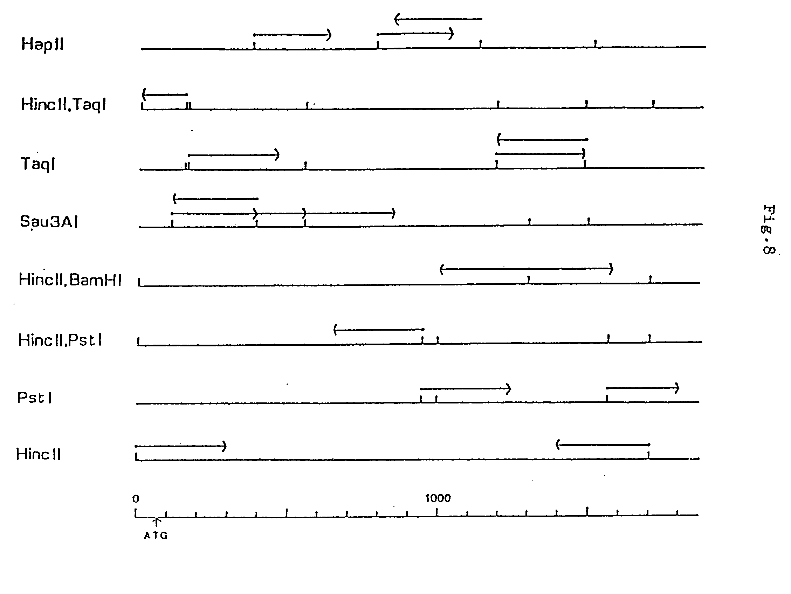

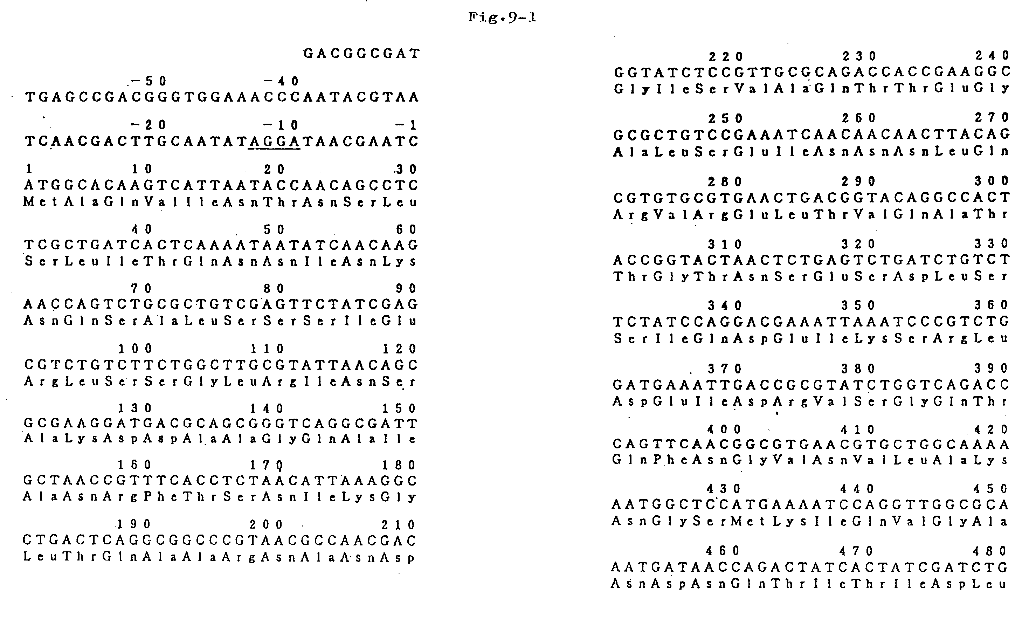

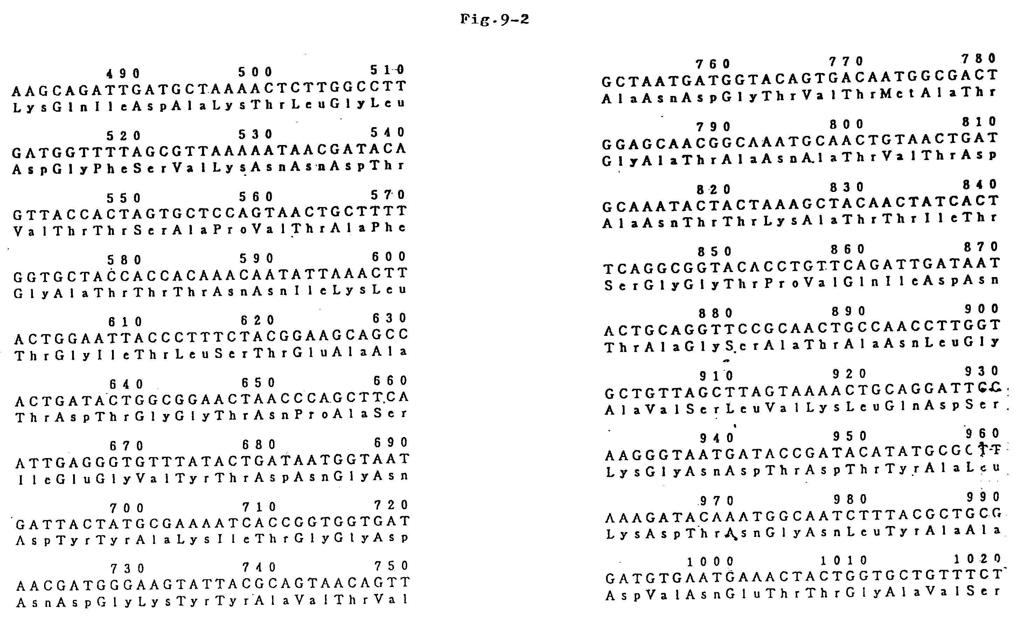

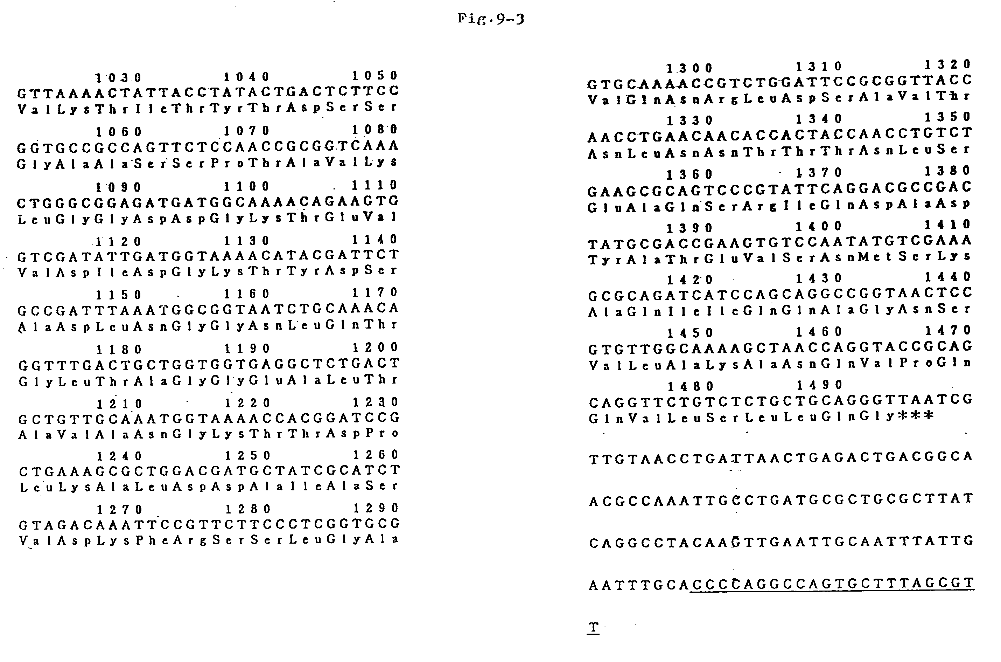

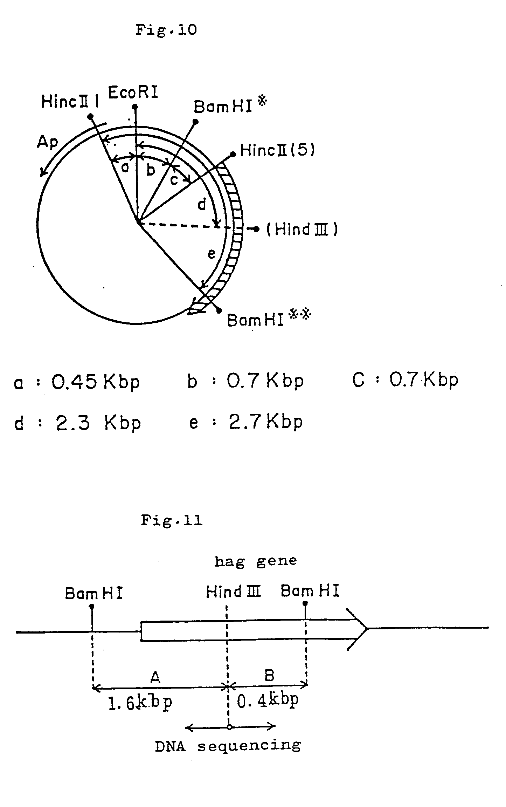

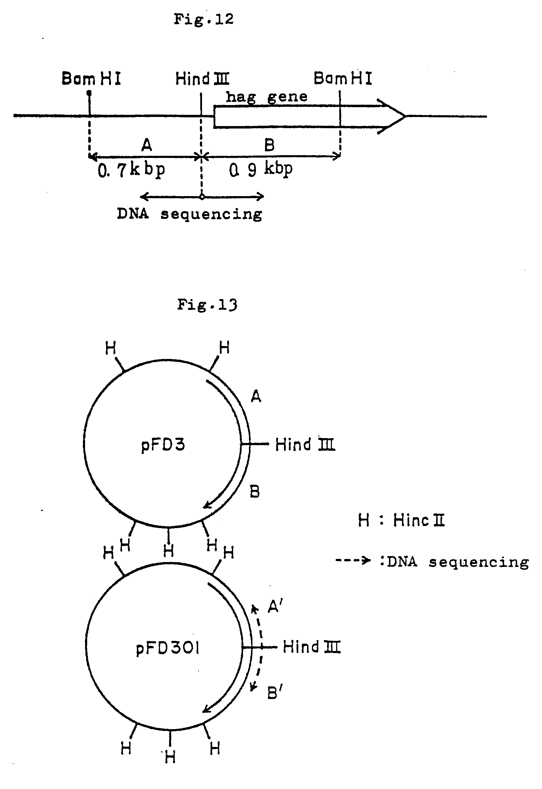

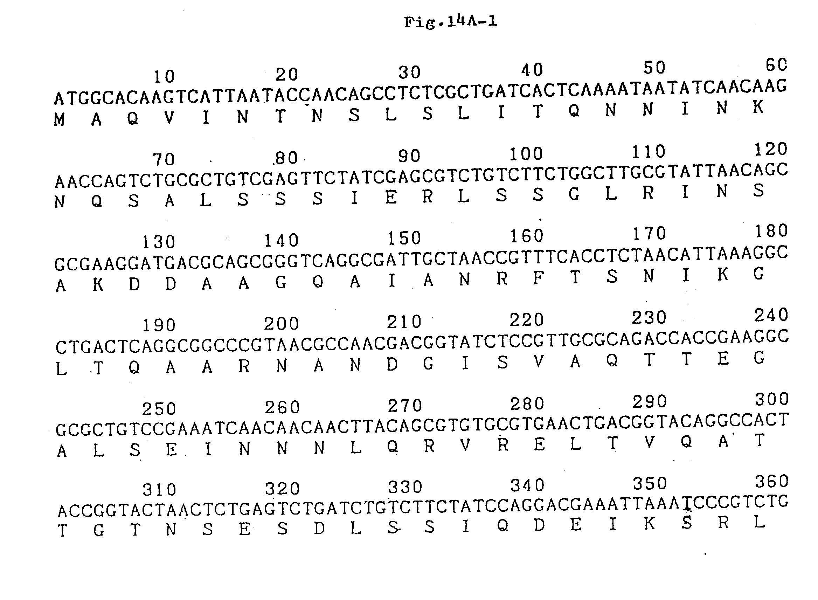

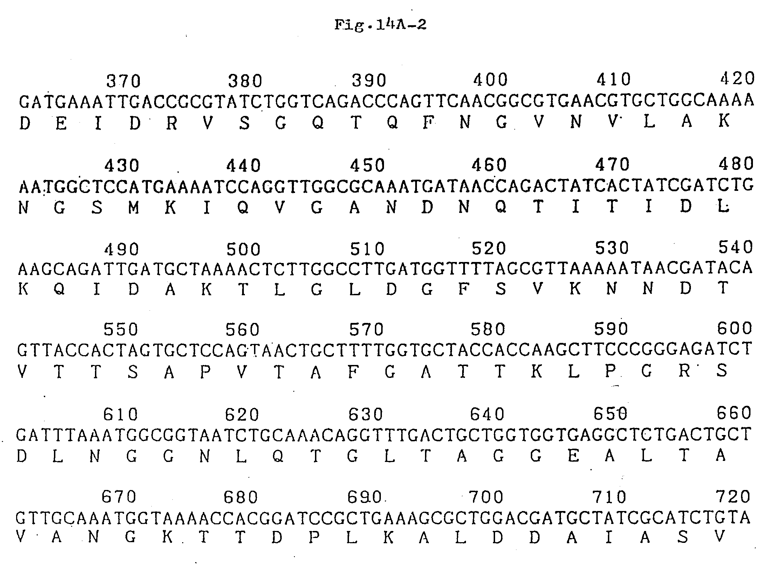

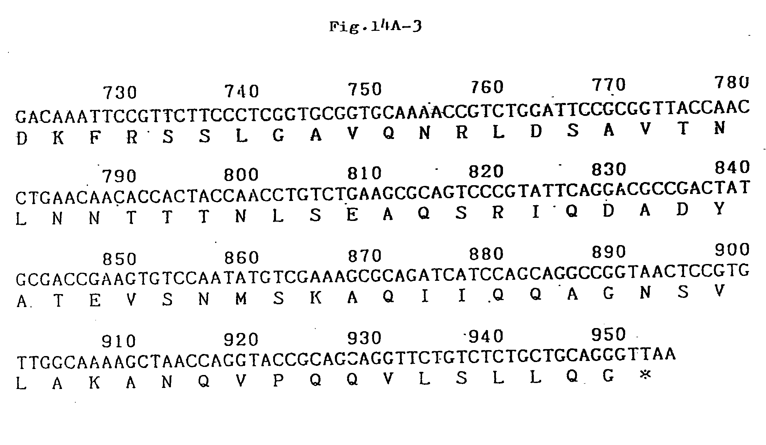

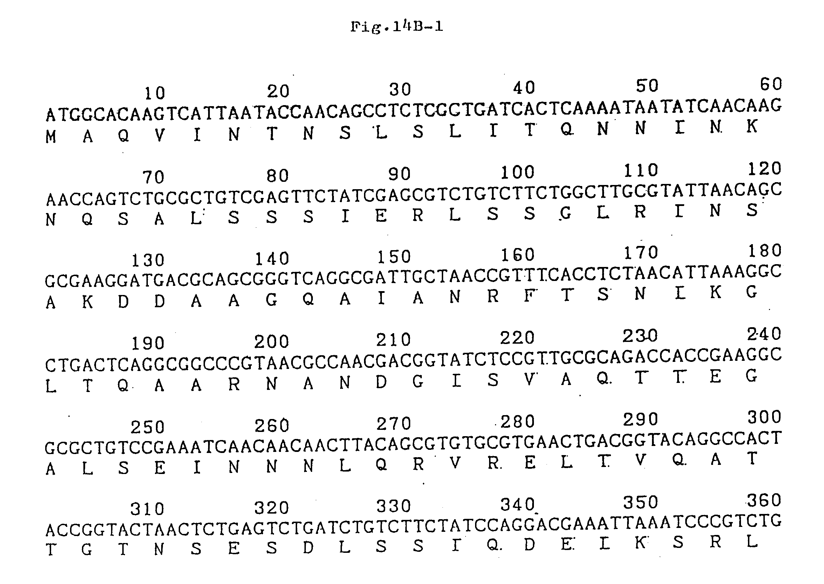

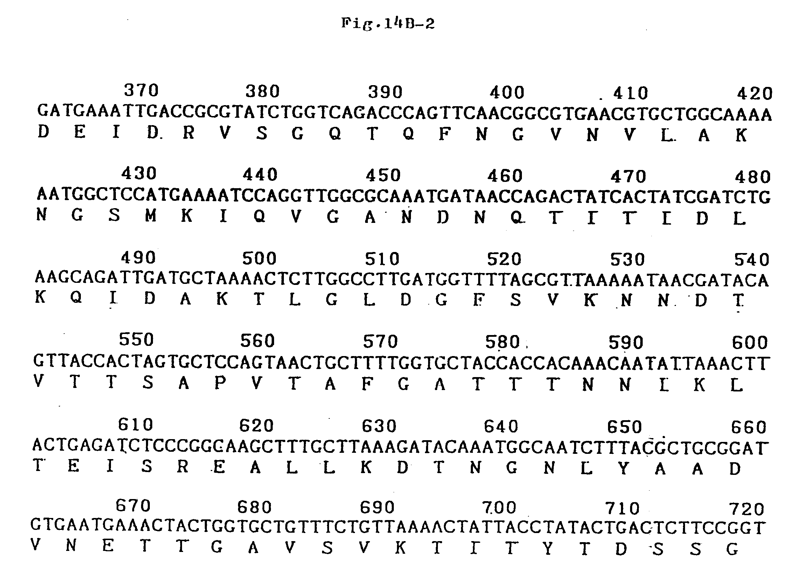

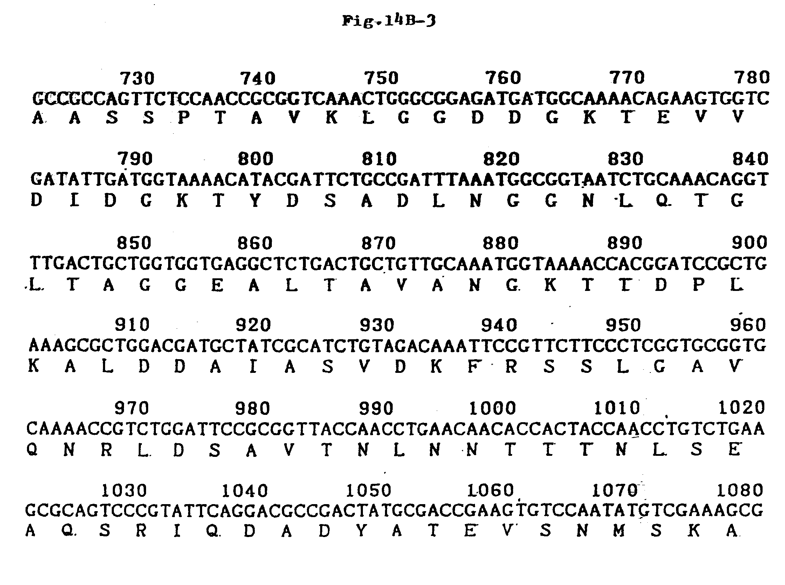

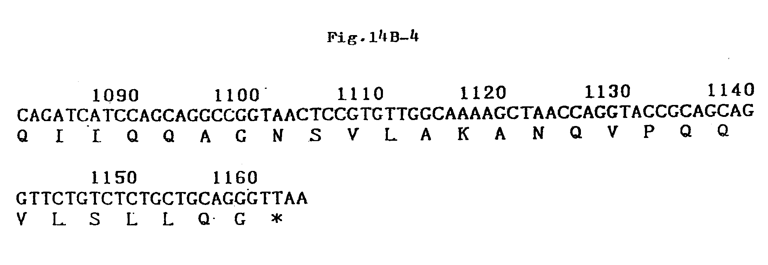

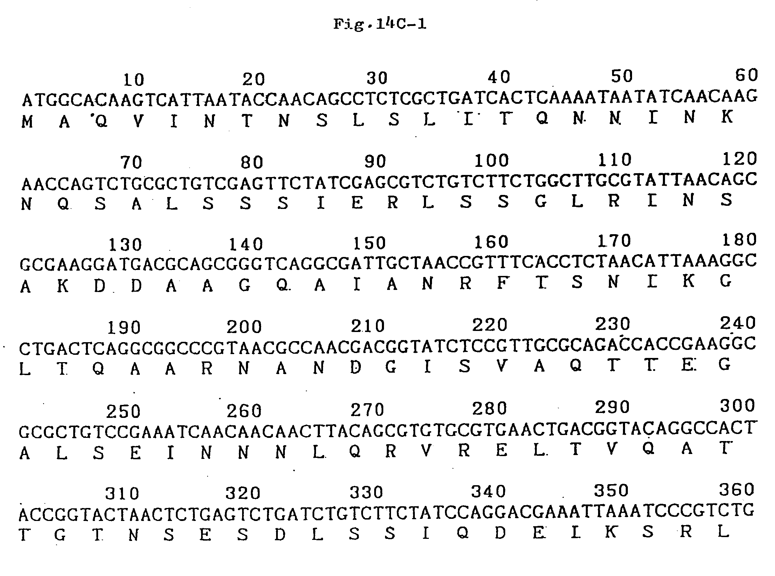

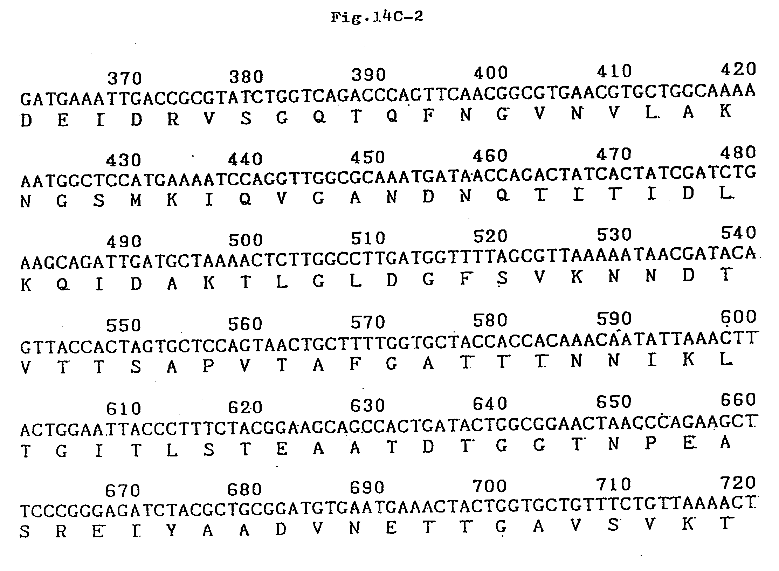

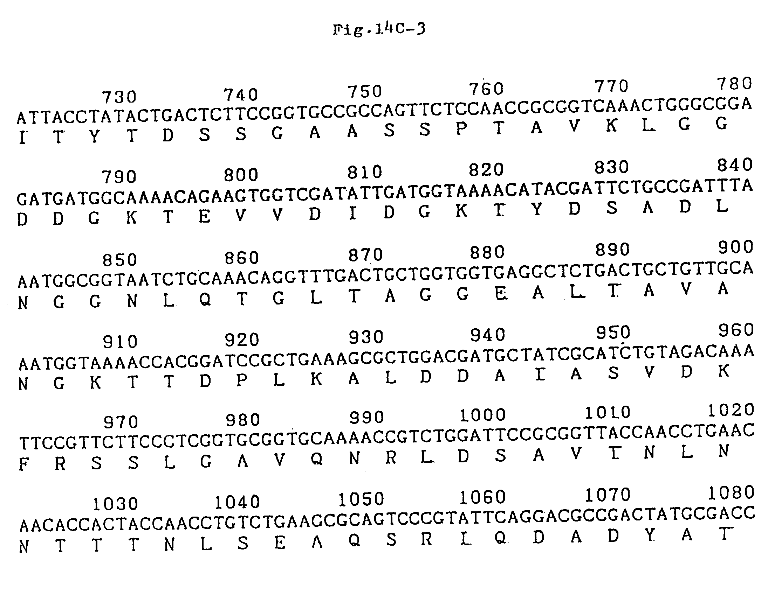

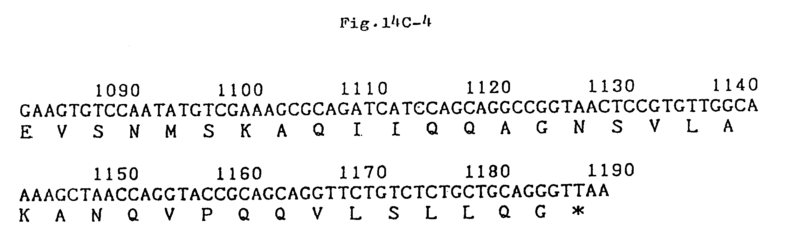

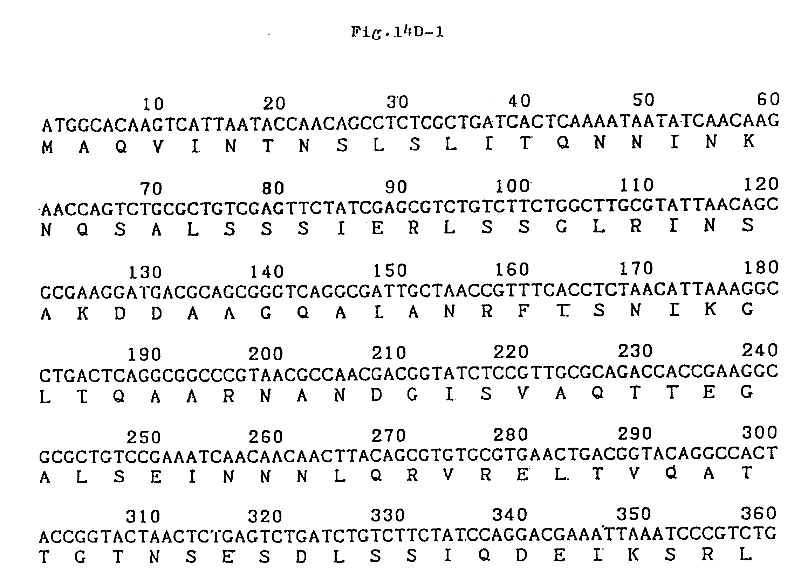

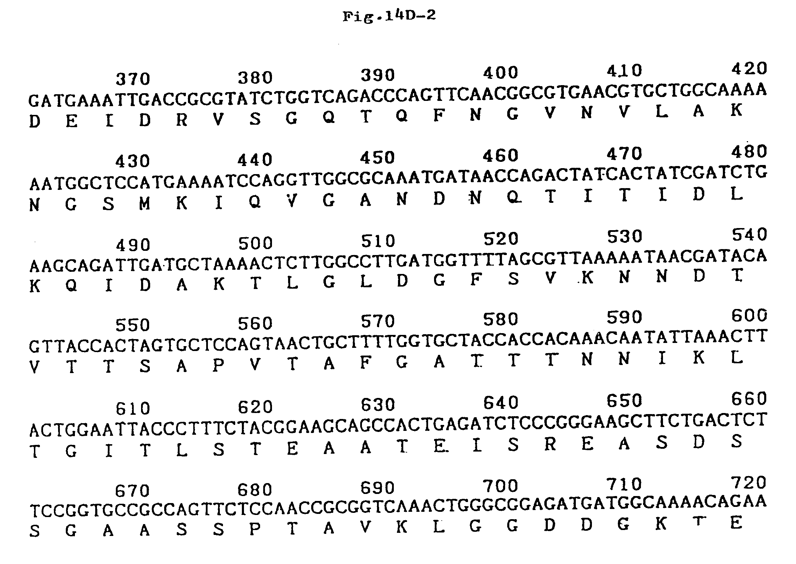

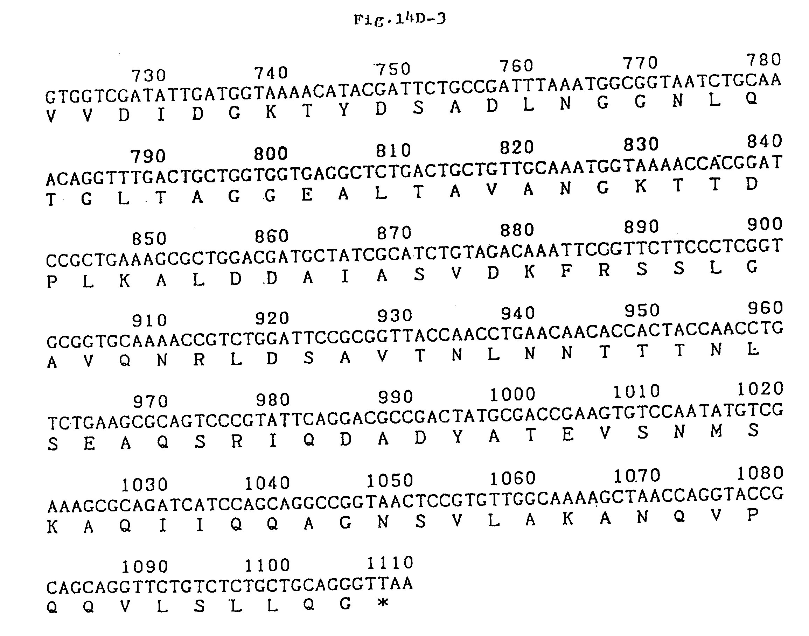

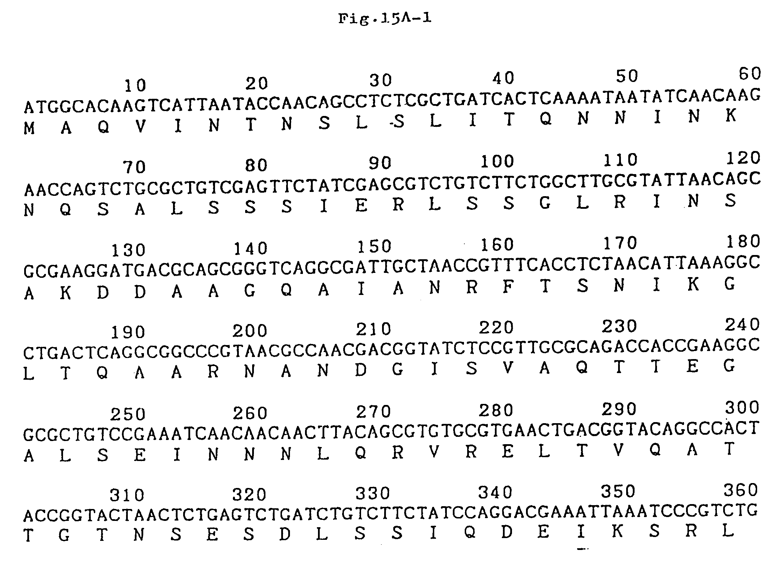

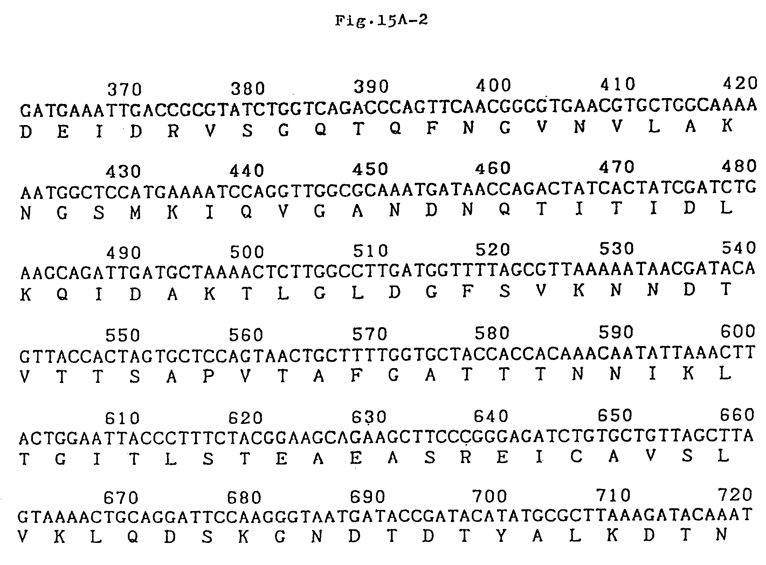

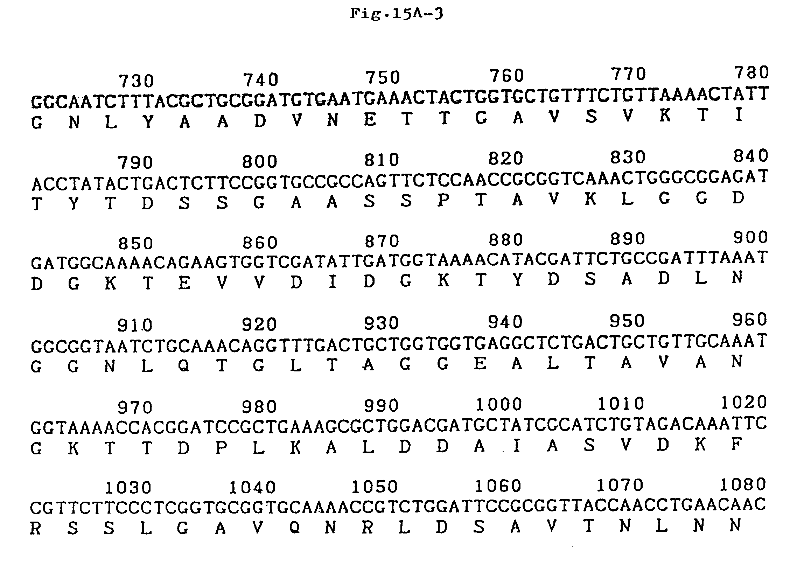

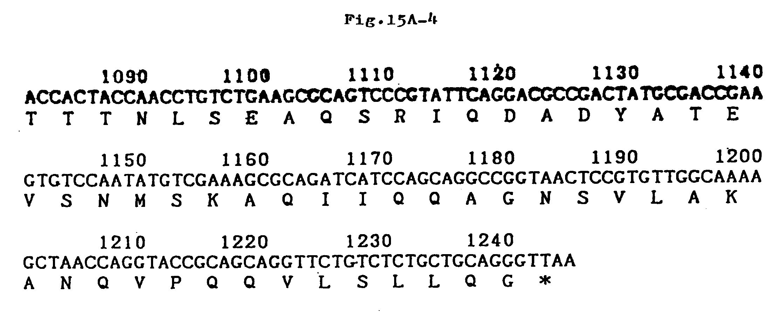

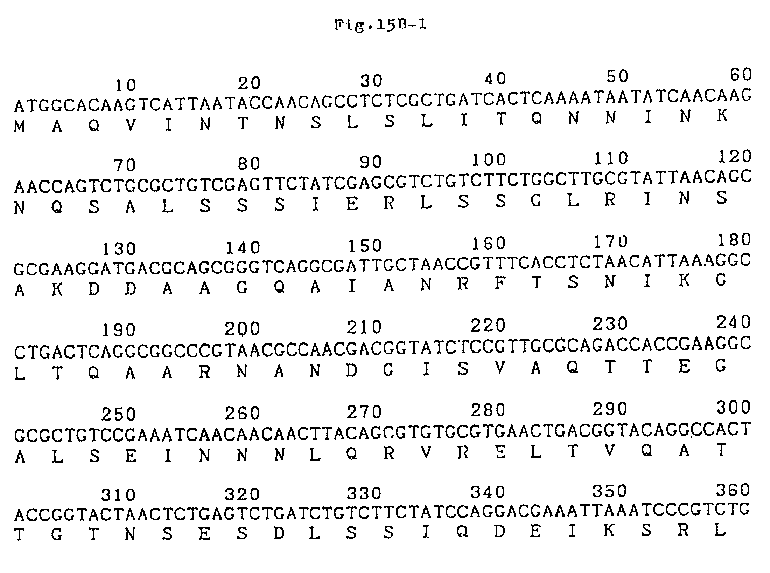

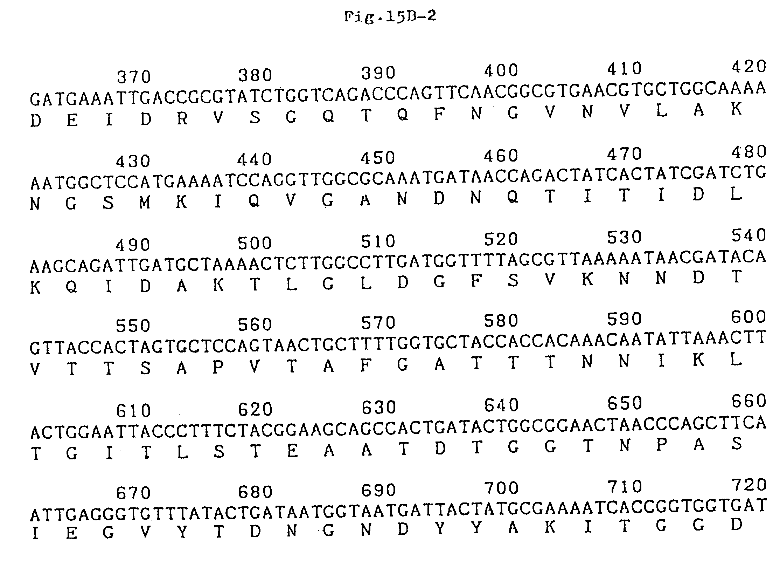

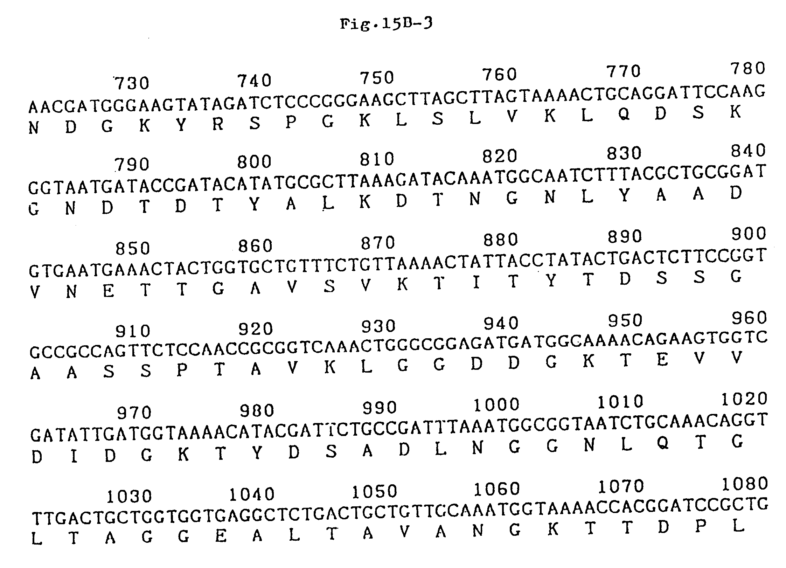

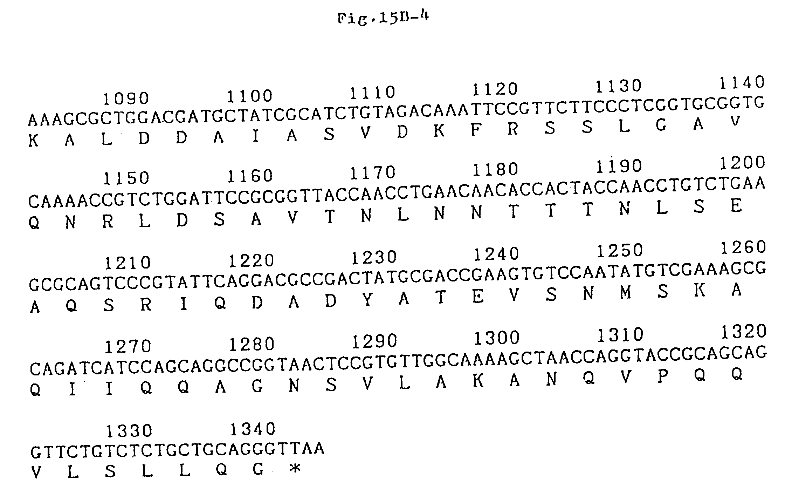

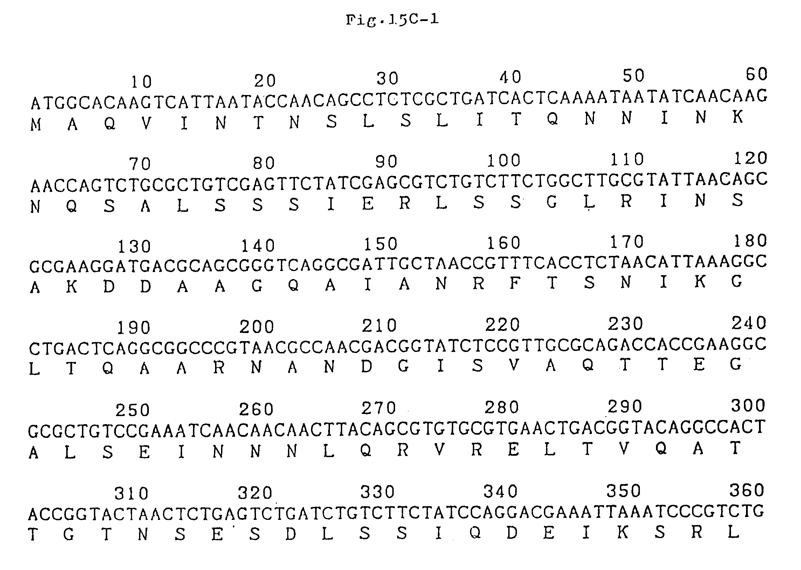

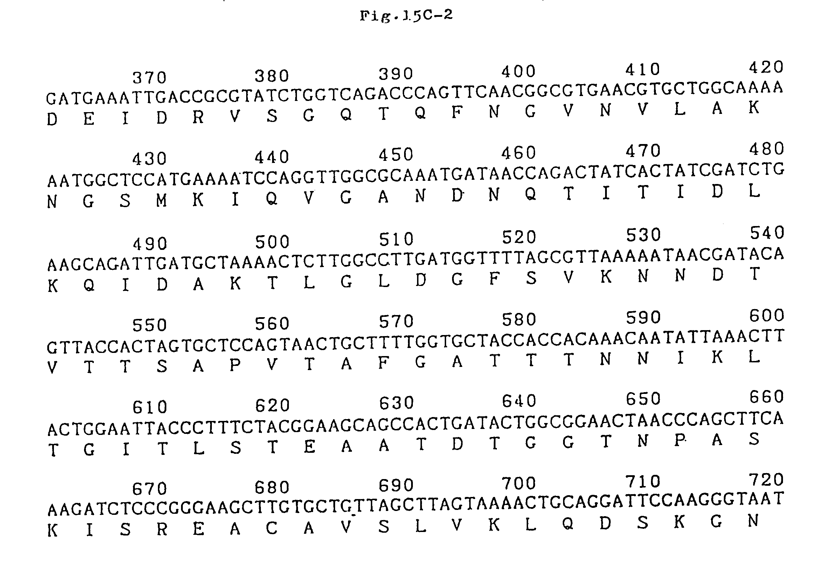

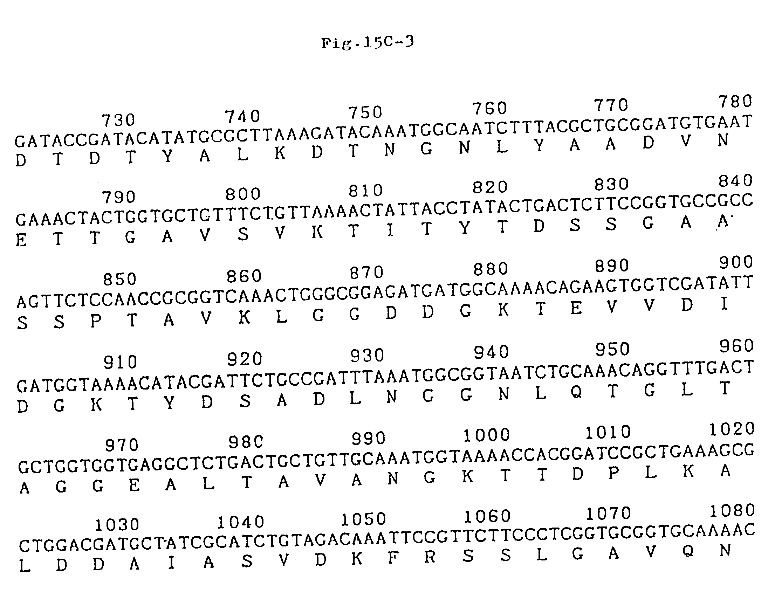

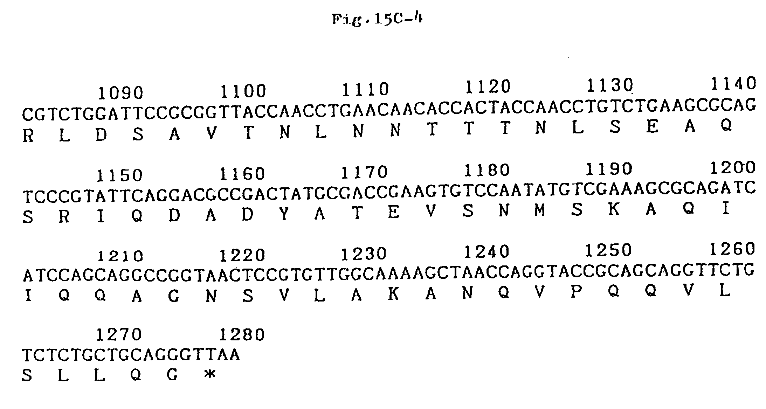

Fig. 1 shows the DNA sequence of the hag gene encoding flagellin of E.coli and the amino acid sequence deduced from the DNA sequence. Fig. 2 shows the result of digestion of λ pfla-H2 with EcoRI and SalI, in which oblique lines indicate a deduced position of the DNA fragment carrying the hag gene derived from the chromosome of E. coli. Fig.3 shows the result of digestion of pBR322/hag9. with EcoRI and Sal I. Fig. 4 shows BamHI restriction sites on pBR322/hag9. Fig. 5 shows pBR322/hag93 lacking fragment B of pBR322/hag9. Fig. 6 shows 5 Hinc II restriction sites on pBR322/hag93. Fig. 7 shows pUC9/hag6. Fig. 8 shows a schedule for sequencing of the hag gene. Fig. 9 shows the whole primary structure of the hag gene determined by DNA sequencing. Fig. 10 shows a brief restriction map of pFD1, pFD2 and pFD3. Fig. 11 shows the DNA sequencing schedule around the HindIII restriction site on pFD1, pFD2 and pFD3. Fig. 12 shows the DNA sequencing schedule around the HindIII restriction site on pHD1. Fig. 13 shows pFD3 and pFD301. Fig. 14 shows sequences of the hag gene carried on pFD301, pFD303, pFD306 and pFD307. Fig. 15 shows sequences of the hag gene carried on pFD311, pFD313 and pFD315.

The present inventors found an excretion system of flagellin when investigating a system in which a target peptide is produced outside of a cell.

One object of the present invention is a DNA having a DNA sequence encoding a flagellin protein lacking central amino acid residues which are unnecessary for excretion in bacteria. The flagellin is obtained by deleting the part of the DNA sequence encoding the central amino acid residues.

A further object of the present invention is a vector having DNA sequence as mentioned above encoding a flagellin protein, capable of expressing the flagellin protein.

In a preferred emodiment of the present invention the DNA sequence is the hag gene encoding a protein lacking the central amino acid residues which are unnecessary for excretion in bacteria. The hag gene was inserted into a vector and then the hag gene in the vector was cut or a part of the hag gene in the vector was deleted and linker DNA was inserted into the resulting spaced DNA coding for a foreign peptide is inserted into the linker DNA in the vector and the resulting vector is introduced into a bacterium. As a result, the foreign peptide is excreted from the bacterium as a peptide fused with flagellin. Under certain conditions the excreted peptide forms flagella, thus facilitating the recovery of the peptide. Moreover, certain foreign peptides which could not be secreted from bacteria by conventional secretion systems may be excreted by the excretion system of the invention.

The DNA having a DNA sequence encoding flagellin can be prepared from all kinds of bacteria that have flagella such as *E*. *coli*, *Bacillus subtilis*, *Proteus* and so on. For example, the hag gene encoding flagellin of *E*. *coli* is prepared in accordance with the method of Kondoh et al (J. Bacteriol. 130, 736-745 (1977)). However, it is easier to use the hag gene already cloned in pBR322 or λ phage.

The present invention is explained with reference to the hag gene but is not restricted to the use of the hag gene since every DNA having a DNA sequence encoding flagellin can be employed in this invention.

A phage or plasmid carrying the hag gene is introduced into a bacterium that cannot form flagella because of a mutation in the hag gene. The bacteria are screened according to their motility. Bacteria having a mutation in the bag gene may be prepared according to the method of Kondoh et al (Genetics 84, 403-421 (1976)). The strain W3623Hfla-am76 prepared by the above method may be used in this invention. If foreign DNA is to be inserted into the hag gene contained in the vector, *E. coli* K-12 C600 r⁻m⁻ hag::Tn10 or *E. coli* K-12 JA11 (deposited with Fermentation Research Institute Agency of Industrial Science and Technology as FERM P-8853 on Jul. 17, 1986 and transferred into the deposition under the Budapest Treaty as FERM BP-1233 on Dec. 4, 1986) which cannot form flagella because of a Kanamycin-resistance gene which is introduced into the hag gene of its chromosome may be used. In the examples of this specification the strain K-12 C600 r⁻m⁻ hag::Tn10 is employed. However, the strain K-12 JA11 deposited with the Fermentation Research Institute Agency of the Industrial Science & Technology can preferably be used for repeating this invention. As can be seen from the following examples, the vectors of this invention can be employed without difficulty by employing the strain K-12 JA11.

Since a strain transformed with the complete hag gene recovers motility, the DNA having the hag gene may be isolated from a strain showing motility. Namely, the DNA having the complete hag gene is prepared by fermenting the strain in a large scale and purifying it by usual centrifugation, removal of protein, ethanol precipitation and so on.

A DNA fragment containing the hag gene is prepared by digesting the DNA with suitable restriction enzymes and-then by inserting it into an appropriate vector. Applicable restriction enzymes depend upon the DNA sequence carrying the hag gene. Preferred restriction enzymes are those which do not cut the hag gene but do cut as much as possible of the unnecessary DNA sequence. Suitable vectors are exemplified below: plasmid vectors such as pN01523, pSC101, pRK353, pRK646, pRK248, pDF41, ColE1, pVH51, pAC105, RSF2124, pCR1, pMB9, pBR313, pBR322, pBR324, pBR325, pBR327, pBR328, pKY2289, pKY2700, pKN80, pKC7, pKB111, pKB158, pKH47, pHSV-106, pKK2333, pMK2004, pACYC1, pACYC184, pUC8, pUC9, pUC12, pUC13, pUC18, pUC19, pAT153, pUR222, pBT11, pJDB207, Homer 111, pHSV-106, ptac 12, ptrpL1, pSV-neo, cloDF13, R6K, F, RI, R6, Entp307, pC194, pE194, pA0501, pUB110, pI127 and so on, phage vectors such as M13, λ gt. λ c, λ gt. λ B, λ WE5 λ C, λ WE5. λ B, λ ZJvir. λ B', λ ALO. λ B, λ WES.Tₛ622 and so on, and vectors constructed from these vectors. Not restricted to only the above vectors, all vectors which can carry the hag gene and can be introduced into a host may be employed in this invention. A vector carrying the hag gene may be minimized and the hag gene may be further transferred into another suitable vector.

A vector prepared as noted above was purified according to a usual method and the whole sequence of the hag gene was determined. The sequencing can be performed by the usual methods such as the method of Maxam and Gilbert, the dideoxynucleotide chain termination method and so on. The whole base sequence of the hag gene is shown in Fig. 1. The amino acid sequence deduced from the base sequence is shown below the base sequence in Fig. 1.

The amino acid residues of the N-terminal and the C-terminal of the flagellin protein of E. coli were analyzed and determined to be Ala-Gln- at the N-terminal and -Gly at the C-terminal, which are identical with the amino acid residues deduced from the base sequence. The experimental data of the amino acid composition are very close to the data calculated from the base sequence.

In order to easily handle a vector prepared as an excretion vector, suitable linkers may be inserted into the hag gene of the vector without destruction of the structure necessary for the excretion of flagellin by such an insertion. Said linkers may provide suitable restriction sites in the hag gene without destroying the excretion function of the flagellin.Preferred is a small number of restriction sites, especially a unique restriction site in the vector. For example, when the hag gene is inserted into pBR322, one or more linkers having a restriction site e.g.for Hind III, Avr II, Bcl I, BstE II, Bgl II , Hpa I , Kpn I , Sac I , Sac II , Sma I, Sst I , Sst II , Xba I , Xho I and Xma I are preferably inserted into the hag gene. Such an insertion of linkers makes it easy to insert a foreign gene into the hag gene.

The part of the hag gene which is obtained by deleting the part which is unnecessary for the excretion of the flagellin protein is utilized in an excretion vector. It was revealed that the flagellin expressed by the hag gene is excreted even if amino acid residues in the central part of the flagellin protein are deleted. Namely, if a vector which carries the hag gene lacing its central part is introduced into a bacterium which is not capable of forming flagella, the bacterium becomes able to form flagella and shows motility. Moreover, when linkers are inserted into the central part of the hag gene contained in a vector and exogenous DNA is inserted into the linker, the vector induces the host to excrete the exogenous protein fused with flagellin and to form flagella.

Since the central part of the hag gene codes for amino acid residues which are responsible for the antigencity of flagellin, the deficiency of the central portion of the hag gene results in a change of the antigencity of flagella. In this invention the vectors with which E.coli is transformed and by which the antigencity, of flagella is changed in such a way that E. coli shows motility on a medium containing anti-flagella antibody, are prepared in the following way: A vector carrying the hag gene is cut at random, digested from the cut site, then linked for recyclization with adding linkers and introduced into E. coli. The vector can induce E. coli to form flagella and the linkers are inserted into the hag gene contained in the vector. Since the DNA is cut and digested at random in this step, several kinds of vectors having similar properties but somewhat different sequences are prepared. The ordinary skilled person in this field can easily predict that vectors can be prepared which are somewhat different from the vector prepared as describe above in the lacking portion of the hag gene in terms of the cut site and the number of bases digested from the cut site. Accordingly, this invention is not restricted to specific vectors disclosed in this specification but includes vectors having the properties as mentioned above as well as vectors prepared according to the method as noted above. This invention reveals that a vector lacking the DNA sequence from bp 683 to 1,143 (561 base pairs) of the hag gene and having a 18mer linker in the lacking portion shows the above-mentioned properties.

If it is not necessary to induce a host to form flagella but only to excrete a fused protein, the lacking portion of the hag gene on the vector may be expanded.

Moreover, adding the pHD1 mutation (see Example 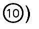 ) to the vectors of this invention is efficient for inducing E. coli to form a large number of flagella.

The abbreviations used in this specification are shown below.
DNA : deoxyribonucleic acid
cDNA : complementary DNA
ccDNA : closed circular DNA
RNA : ribonucleic acid
mRNA : messenger RNA
A : adenine
T : thymine
G : guanine
C : cytosine
ATP : adenosine triphosphate
TTP : thymidine triphosphate
GTP : guanosine triphosphate
CTP : cytidine triphosphate
dATP : deoxyadenosine triphosphate
dTTP : deoxythymidine triphosphate
dGTP : deoxyguanosine triphosphate
dCTP : deoxycytidine triphosphate
ddATP : dideoxyadenosine triphosphate
ddTTP : dideoxythymidine triphosphate
ddGTP : dideoxyguanosine triphosphate
ddCTP : dideoxycytidine triphosphate
DTT : dithiothreitol
Ala or A : alanine
Arg or R : arginine
Asn or N : asparagine
Asp or D : asparatic acid
Cys or C : cysteine
Gln or Q : glutamine
Glu or E : glutamic acid
Gly or G : glycine
His or H : histidine
Ile or I : isoleucine
Leu or L : leucine
Lys or K : lysine
Met or M : methionine
Phe or F : phenylalanine
Pro or P : proline
Ser or S : serine
Thr or T : threonine
Trp or W : tryptophan
Tyr or Y : tyrosine
Val or V : valine

### Example

### ① Recloning of the hag gene in pBR322

### (A) Preparation of DNA of phage λ pfla-H2 having hag gene

### (a) Identification of phage λ pfla-H2

*E. coli* K-12 W3623Hfla-am76 (deposited with Fermentation Research Institute Agency of Industrial Science and Technology as FERM P-8619 on Jan. 25, 1986 and transferred into the deposition under the Budapest Treaty as FERM BP-1231 on Dec. 4, 1986; Genetics 84, 403-421 (1976)), which has a mutation in the hag gene and forms no flagellum, is incubated overnight at 37°C with shaking in trypton broth for phage λ (comprising 1% bactotrypton (DIFCO), 0.25% sodium chloride and 0.0005% thiamine hydrochloride, pH 7.0 before sterilization). To the incubated broth (0.1ml) 0.1ml of trypton broth containing several hundreds of phage λpfla-H2 carrying the hag gene of E. coli K-12 KH552 (J.Bacteriol. 130, 736-745 (1977)) is added. Then 3.0ml of trypton broth supplemented with 0.6% agar is added at 47°C and the resultant is put upon a trypton plate supplemented with 1.2% agar. After overnight incubation at 37°C, several hundreds of plaques are formed.

Each plaque is transferred by a sterilized toothpick to a medium for the motility test (above-mentioned trypton broth supplemented with 0.3% agar), and incubated at 37°C for 7-8 hr. The bacteria infected by the phage capable of completely transforming the hag gene form colonies of a motile area of 3-4cm in diameter. The media containing plaques forming such a colony are turned upside down. A few drops of chloroform are dropped to the lids and the mixture is allowed to stand at 37°C for 10 min for sterilization.

### (b) Incubation of phage λ pfla-H2

Said phage λ pfla-H2 is suspended in 0.1ml of the trypton broth as noted above, using a sterilized toothpick. To this suspension 0.1ml of E. coli K-12 C600 incubated overnight in trypton broth and then 3ml of trypton broth (46°C) supplemented with 0.6% agar is added. The resultant is put over trypton broth supplemented with 1.2% agar and incubated at 37°C for 5-6 hr. After the strain C600 is almost lysed by the phage λ pfla-H2, 5ml of trypton broth is added thereto and the mixture is incubated for 20-30 min. After a few drops of chloroform are added thereto, the 0.6% agar put over is broken, transferred into a centrifuge tube together with 5ml of the broth added later and centrifuged at 3,000 r.p.m. for 10 min. To the supernatant a few drops of chloroform are added and the mixture is incubated at 37°C.for 5 min for sterilization to give a seed phage solution of phage λ pfla-H2. The seed phage solution is diluted 10⁶-10⁷ times with trypton broth, 0.05ml of which is mixed with 0.1ml of C600 incubated overnight in trypton broth. To the resultant 3ml of trypton broth (46°C) supplemented with 0.6%agar is added and the mixture is put over trypton broth supplemented with 1.2% agar. The number of phages per 1ml is calculated from the number of plaques emerged after overnight incubation at 37 °C. The above-mentioned dilute seed phage solution (0.1ml) containing 2 x 10⁷ phages is mixed with 0.1ml of the culture of C600 incubated overnight in trypton broth supplemented with 0.2% maltose and incubated at 37°C for 10 min. Trypton broth (46°C) supplemented with 0.6% agar (3ml) is added thereto and the mixture is put over a trypton plate containing 1.2% agar. Fifty plates as noted above are prepared. The plates are incubated at 37°C for 5-6 hr. After most of the strain C600 is lysed by the phage λpfla-H2, 5ml of trypton broth is added to each plate, followed by further incubation for 20-30 min. A few drops of chloroform are added thereto and then the 0 6% agar put over is broken, transferred into a centrifuge tube together with 5ml of trypton broth and centrifuged at 3,000 r.p.m. for 10 min. The supernatant is recovered to give about 350 ml of the culture solution of the phage.

### (c) Purification of phage λ pfla-H2

The above-mentioned phage culture solution is ultra-centrifuged at 25,000 r.p.m. at 4°C for 90 min. (Beckman L8-55 ultracentrifuge, #30 rotor). After the supernatant is removed, a small quantity of TM buffer solution (10mM Tris-HCl (pH8.0), 10mM magnesium sulfate) is added to the precipitate. To this suspension 1.7 ml of TM buffer solution saturated with cesium chloride is added at 4°C per 1.3ml of the suspension, followed by centrifugation at 23,000 r.p.m. at 15°C for 40 hr (Beckman L8-55, SW41 rotor). The phage gathering around the center of the centrifuge tube forms a visible band. The portion of the band (c.a. 1ml) is drawn out of the centrifuge tube with an injector and dialyzed overnight at 4°C against TM buffer solution (10mM Tris-HCl (pH 8.0), 1mM Na₂EDTA).

### (d) Preparation of DNA of λ pfla-H2

To one part of the above suspension of phage λ pfla-H2 0.1 parts of 10 × SSC buffer solution (1.5M sodium chloride and 0.15M sodium citrate), 0.02 parts of 0.5M Na₂EDTA and 0.01 parts of 20% sodium dodecyl sulfate (SDS) are added. The suspension is incubated at 37°C for 10 min. Then the same volume of TE buffer solution saturated with phenol is added thereto. Phenol extraction of DNA is carried out with a rotary extractor at 60 r.p.m. for 30 min. The content is transferred into a centrifuge tube and centrifuged at 8,000 r.p.m. at 20°C with a high speed refrigerated centrifuge. The supernatant is transferred into a dialysis tube and dialyzed overnight against TE buffer solution. The dialysate is transferred into SW41 polymer centrifuge tube (Beckman) and 0.1 volumes of 3M sodium acetate and then 2 volumes of cold ethanol are added thereto, followed by incubation overnight at -20°C. The resultant is centrifuged at 300,000 r.p.m. at 4°C for 60 min with a centrifuge (Beckman) to recover the precipitate. The precipitate is dissolved in 400 µl of TE buffer solution and determined to be about 300 µg of the purified DNA of λ pfla-H2 from an absorbance at 260nm.

### (B) Presumption of the position of the hag gene on λ pfla-H2 DNA

### (a) Digestion of λ pfla-H2 DNA with EcoRI

λ pfla-H2 DNA is digested with EcoRI. To a reaction mixture (20 µl) containing 500ng of λ pfla-H2 DNA, 50mM Tris-HCl (pH7.5), 7mM MgCl₂, 100mM NaCl, 7mM 2-mercaptoethanol and 0.01% bovine serum albumin, which is heated to 37°C, 5 units/1µl of EcoRI (Takara Shuzo Co, Ltd.) are added, followed by incubation at 37°C for 30 min. A solution (2 µl) for inhibiting enzyme reaction (a mixture of 50% glycerol, 1% SDS and 0.02% bromophenol blue) is adds thereto so as to inhibit the reaction. The reaction mixture (5 µl) is charged to a 0.8% agarose slabgel electrophoresis. Using submarine agarose electrophoresis system (Marysol Industry Co., Ltd.) and a electrophoresis buffer solution containing 40mM Tris-HCl (pH8.1), SmM sodium acetate and 1mM Na₂EDTA, the electrophoresis is performed at constant voltage (80V) for 30-60 min. After the electrophoresis the gel is soaked in the above buffer solution supplemented with 0.5µg/ml of ethidium bromide for about 10 min and irradiated with an ultraviolet lamp (280nm) to detect the DNA hand. As a result, it is determined that λ pfla-H2 DNA is digested with EcoRI into 21.3, 17.0, 5.82 and 3.54 kb fragments.When the DNA of bacteriophage λ CI857S7 (Takara Shuzo Co., Ltd.) is digested with EcoRI in the same manner, fragments of 21.3, 5.82 and 3.54 kb are detected but the fragment of 17.0 kb is not seen. Accordingly, it is deduced that the fragment of 17.0 kb contains a DNA fragment containing hag gene of E. coli.

### (b) Digestion of λ pfla-H2 DNA with SalI

To a mixture containing 10mM Tris-HCl (pH7.5), 7mM MgCl₂, 175mM NaCl, 0.2mM EDTA, 7mM 2-mercaptoethanol and 0.01% bovine serum albumin 500ng of λpfla-H2 DNA is added, the DNA is digested with Sal I in the same manner as noted above and applied to an agarose gel electrophoresis. The λ pfla-H2 DNA is digested into 32.7 and 15.3 kb fragments by Sal I. Since a fragment of 15.3 kb is generated also in a digestion of λ CI857S7 DNA with Sal I, it is deduced that the fragment of 32.7kb carries a DNA fragment containing the hag gene.

Fig. 2 shows the result of a digestion of λ pfla-H2 with EcoRI (①-B-a) and Sal I (①-B-b) and the deduced position of a DNA fragment derived from chromosome carrying the hag gene of *E. coli*.

### (C) Cloning in vector pBR322

λ pfla-H2 DNA (2.5µg) is digested with EcoRI in 100µl of a reaction mixture according to ①-B-a. After inhibiting the reaction by heating the mixture to 70°C for 5 min, 10µl of 3M sodium acetate and 220µl of cold ethanol are added thereto, followed by incubation overnight at -20°C. After 10 min centrifugation (Microfuge, Beckman) the supernatant is removed and the precipitate is dissolved in 70µl of TE buffer solution.

To the resultant (λ pfla-H2 DNA digested with EcoRI) a reaction mixture and Sal I (total volume 100µl) are added for digestion in a similar manner to ①-B-b. After the reaction, ethanol precipitaion is performed in the same way as noted above and the precipitate is dissolved in 50µl of TE buffer solution.

Besides, to a reaction mixture (144µl) prepared in a similar manner to ①-B-a, which contains 2µg of pBR322 cc-DNA (Takara Shuzo Co., Ltd), 24 units/4µl of EcoRI and 16 units per 2µl of HindIII are added and the reaction mixture is incubated at 37°C for 30 min. After inhibiting the reaction by heating the mixture to 70°C for 5 min, ethanol precipitation is performed and the precipitate is dissolved in 100 µl of TE buffer solution. Then, the resultant is digested with Sal I, followed by ethanol preciptiation. The precipitate is dissolved in 40µl of TE buffer solution.

To 98µl of T4 DNA ligase reaction mixture (66mM Tris-HCl (pH7.6), 6.6mM MgCl₂, 10mM DTT and 1mM ATP) containing 2.5µg of λ pfla-H2 DNA digested with EcoRI and Sal I and 250µg of pBR322 digested with EcoRI, Sal I and Hind III 0.2 units/2µl of T4 DNA ligase (Takara Shuzo Co., Ltd., Lot301) are added and the reaction mixture is incubated at 22-23°C for 6 hr. After ethanol precipitation the precipitate is dissolved in 20µl of TE buffer solution.

The competent W3623H fla-am76 strain (210µl) is mixed with 250ng/20µl of the above DNA for infection. The mixture is inoculated into LB broth (pH 7.0-7.2) containing 1% Bacto-Trypton(Difco), 0.5% yeast extract and 0.5% NaCl and incubated at 37°C for 1 hr with shaking. 0.1ml of the resultant is spread on a LB plate containing 0.005% ampicillin and 1.5% agar. After overnight incubation at 37°C, an ampicillin-resistant strain growing up is inoculated into a medium for the motility test containing 0.005% ampicillin. Four of 180 colonies tested form a motile area. One of these 4 colonies is purified twice in LB agar medium containing 50µg/ml of ampicillin to establish strain W3623Hfla-am76 ( pBR322/hag9), which has been deposited as FERM P-8620 with the Fermentation Research Institute Agency of the Industrial Science & Technology since January 25, 1986 and transferred into the deposition under the Budapest Treaty as FERM BP-1232 since December 4, 1986.

### (D) Investigation of the structure of the recombinant plasmid pBR322/hag 9

### (a) Purification of pBR322/hag9 cc-DNA

Strain W3623Hfla-am76(pBR322/hag9) is inoculated in 5ml of LB broth containing 50µg/ml of ampicillin and incubated overnight at 37°C with shaking. This overnight culture is transplanted in 1L of LB broth containing 50µg/ml of ampicillin, incubated overnight at 37°C with shaking and centrifuged at 5,000 r.p.m. at 4°C for 10 min to recover the bacteria.

The recovered microorganisms are suspended in 20ml of solution I (50mM glucose, 25mM Tris-HCl (pH 8.0) and 10mM EDTA), in which 100mg (powder) of lysozyme (Sigma) is dissolved. The mixture is allowed to stand at room temperature for 10 min with occasional stirring and 40ml of solution II (0.2N sodium hydroxide and 1% sodium dodecyl sulfate) is gradually mixed therewith, followed by an incubation in ice for 10 min. Then, the mixture, to which 30ml of ice-cooled 5M potassium acetate (pH 4.8) is added, is allowed to stand in ice for 10 min. After centrifugation at 10,000 r.p.m. at 4°C for 30 min, the supernatant is recovered in a 100ml-measuring cylinder to measure the volume and transferred to a centrifuge tube, mixed with 0.6 parts of isopropanol and incubated at room temperature for 15 min. After centrifuging at 10,000 r.p.m. at 15°C for 30 min and removing the supernatant, 140ml of 70% ethanol is added to the precipitate. After centrifuging the resulting solution at 10,000 r.p.m. at 4°C for 30 min and removing the supernatant, the precipitate is almost dried in a vacuum desiccator and then dissolved in TE buffer solution. The solution is transferred into a 50ml-measuring cylinder to adjust the volume to 30ml, in which 0.3ml of 10% sodium dodecyl sarcosinate, 2ml of 10mg/ml ethidium bromide and then 33.915g of cesium chloride are dissolved. After centrifuging at 36,000 r.p.m. at 15°C for 40 hr (L8-55 ultracentrifuge, VTi 50 rotor, Beckman), 2 fluoresent bands are observed under an ultraviolet lamp. The lower band is drawn from the side of the centrifuge tube with a syringe. Then the content of the syringe is applied to ultracentrifugation at 36,000 r.p.m. at 15°C for 20 hr (Beckman L8-55 ultracentrifuge, VTi 65 rotor). In the same way as mentioned above a lower band which shows fluoresence under an ultraviolet lamp is drawn with a syringe and transferred to a test tube. Ethidium bromide is removed by extracting 3 times with isopropanol saturated with cesium chloride. The water phase is dialyzed overnight against TE buffer solution.

The dialyzed sample is put in a tube for SW 50.1 rotor (Beckman), mixed with 0.1 parts of 3M sodium acetate and 2 parts of ethanol and incubated overnight at -20°C (ethanol precipitation).

After centrifuging the resulting solution at 30,000 r.p.m. at 4°C for 30 min (Beckman L8-55 ultracentrifuge) and removing the supernatant, the precipitate is slightly dried in a vacuum desiccator, dissolved in 0.6ml of TE buffer solution and incubated overnight at 4°C. RNase (Sigma) heated to 96°C for 10 min is added thereto to a final concentration of 10µg/ml and the solution is incubated at room temperature for an hour.

4ml of 1M sodium chloride-TE buffer solution are poured into a tube for a SW 50.1 rotor. The above sample is put over it and centrifuged at 40,000 r.p.m. for 6 hr. The supernatant is removed and the precipitate is dissolved in 0.2ml of TE buffer solution. An aliquot is diluted and the DNA concentration is calculated from the absorbance at 260nm.

### (b) Digestion of pBR322/hag9 cc-DNA with EcoRI and Sal I

In a similar way to ①-B-a and ①-B-b, pBR322/hag9 cc-DNA is digested with EcoRI and Sal I and applied to agarose gel, electrophoresis to observe that the cc-DNA alters to linear DNA, which means that the cc-DNA has an unique restriction site for EcoRI and Sal I .pBR322/hag9 DNA is digested with EcoRI and the reaction is inhibited by heating to 70°C. Successively, the resultant is digested with Sal I and the reaction is inhibited by heating to 70°C. The resultant is applied to agarose gel electrophoresis to observe about 7.5kb and about 3.7kb fragments (λ Cl857S7 DNA digested with Hind III is used as DNA size marker). The about 7.5kb and about 3 .7kb fragments are derived from λ pfla-H2 DNA and pBR322, respectively (see Fig.3).

### (c) Digestion of pBR322/hag9 cc-DNA with BamHI

To 10µl of a reaction mixture coutaining 10mM Tris-HCl (pH8.0), 7mM magnesium chloride, 100mM sodium chloride, 2mM 2-mercaptoethanol, 0. 01% bovine serum albumin and 1µg of pBR322/hag9 cc-DNA 10 units/µl of BamHI are added and the reaction is performed in a similar way to digestion with EcoRI in ①-B-a. The resultant is applied to agarose gel electrophoresis. λ Cl857S7 DNA digested with Hind III is used as DNA size marker. As a result, pBR322/hag9 cc-DNA is digested with BamHI into about 5.7, 3.5 and 1.85kb fragments.

Then, BamHI restriction site on pBR322/hag9 is determined by the following manner; pBR322/hag9 DNA completely digested with EcoRI is incompletely digested with BamHI and pBR322/hag9 DNA completely digested with Sal I is incompletely digested with BamHI. Firstly, 1µg of pBR322/hag9 cc-DNA is digested in a test tube with EcoRI or Sal I according to the method in ①-B-a and ①-B-b. The reaction is performed at 37°C for an hour and inhibited by heating to 70°C for 5 min.

Then, 1 unit/µl of BamHI is added to each test tube and 5µl aliquots are drawn 2 min and 5 min later, to which a mixture for inhibiting the reaction containing 50% glycerol, 1% SDS and 0.02% bromophenol blue is added. To a remained reaction mixture (11µl) 1 unit/µl of BamHI is added 9 min later and 5µl aliquots are drawn 10 min and 20 min later, to which the mixture for inhibiting the reaction (0.5µl) is added. Each sample is subjected to 0.8% agarose gel electrophoresis to determine the size of the produced fragments. λ Cl857S7 DNA digested with Hind III is used as DNA size marker. In a sample completely digested with EcoRI and with BamHI only for 20 min fragments A (5.4kb), B (3.9kb), C (1.95kb) and D (0.73kb) are observed. In a sample completely digested with EcoRI and with BamHI only for 2 min or 5 min fragments corresponding to fragments D, C, B, B+D, A, B+C, B+C+D, A+C, A+B+C and A+B+C+ D according to size are observed. In a sample completely digested with Sal I and with BamHI only for 20 min fragments a (4.7kb), b (3.8kb), c (1.95kb) and d (1.26kb) are observed. In a sample completely digested with Sal I and with BamHI only for 2 min or 5 min fragments coresponding to fragments d, c, c+d, b, a, b+c, b+c+d, a+b, a+b+c, a+b+c+d according to size are observed. Fig, 4 shows BamHI restriction sites on pBR322/hag9.

### (E) Construction of recombinant plasmid pBR322/hag93

To 56 µl of a reaction mixture containing 10mM Tris-HCl (pH 8.0), 7mM magnesium chloride, 100mM sodium chloride, 2mM 2-mercaptoethanol and 0.01% bovine serum albumin 10µg of pBR322/hag9 cc-DNA in 30µl of TE buffer solution and then 40 units of BamHI (4µl) are added and the reaction mixture is incubated at 37°C for 2hr. The resuitant is mixed with 60µl of phenol and chloroform (1:1) and centrifuged with Microfuge to recover the supernatant. To this supernatant again 60µl of phenol and chloroform are added and centrifuged with Microfuge. The supernatant is well mixed with 60µl of chloroform and centrifuged with Microfuge to recover the supernatant. Again, chloroform is added to the supernatant and centrifuged with Microfuge to recover the supernatant. To this supernatant 0.1 parts of 3M sodium acetate and 2 parts of ethanol are added and the mixture is incubated overnight at -20°C. After centrifugation with Microfuge, the precipitate is dissolved in 11µl of TE buffer solution.

DNA linking reaction is achieved as follows. To 11µl of the above sample solution 4µl of 250mM Tris-HCl (pH 7.6) containing 33mM magnesium chloride, 2µl of 100mM ATP, 2µl of 100mM DTT and then 1 unit/ml of T4 DNA ligase (Takara Shuzo Co., Ltd. , Code No. 2010B, Lot No. 301) are added and the mixture is incubated at 13-15°C for 6 hr.

Strain W3623Hfla⁻-hag76 is transformed with 10µl of the above reacted mixture to form 200 colonies on LB agar medium containing 50µg/ml of ampicillin, which are transplanted in the medium for the motility test containing 50µg/ml of ampicillin to give 14 strains showing motility. Plasmid DNA carried by the 14 strains is digested with BamHI according to what is called rapid plasmid DNA analysis*¹. A plasmid lacking fragment B of pBR322/hag9 (see Fig. 4) is found out and named pBR322/hag93 (see Fig.5).
*1 Rapid Plasmid DNA Analysis

A colony is transplanted in LB broth containing 50µg/ml of ampicillin and incubated overnight at 37°C. An 1.5ml aliquot is transferred into an eppendorf tube and centrifuged with Microfuge for 1 min. The supernatant is removed as far as possible and the precipitate is suspended in 100µl of solution I (50mM glucose, 10mM EDTA-Na₂, 25mM Tris-HCl (pH 8.0) and 4mg/ml lysozyme (Sigma)). After incubation at room temperature for 5 min, 200µl of solution II (0.2N sodium hydroxide and 1% SDS) is gradually added thereto and the suspension is incubated in ice (0°C) for 5 min. Then, 150µl of ice-chilled 3M potassium acetate (adjusted to pH 4.8 with gracial acetic acid) is well mixed therewith. After an incubation at 0°C for 5 min, the resulting mixture is centrifuged at 4°C with Microfuge and the supernatant is transferred into another eppendorf tube. The same volume of phenol and chloroform (1:1) is mixed therewith and the mixture is centrifuged with Microfuge for 2 min. The resulting supernatant is transferred into another eppendorf tube. After mixing with 2 parts of ethanol, it is incubated at room temperature for 2 min. After the centrifugation for 5 min with Microfuge and removing the supernatant, a solution remaining on the inner wall of the tube is removed with paper as far as possible. To the tube is added 1ml of 70% ethanol, mixed well and centrifuged with Microfuge for 5 min. The supernatant is completely removed and the precipitate is dried in a vacuum desiccator and dissolved in 50µl Of TE buffer solution containing 20µl/ml of RNase (heated to 95°C for 10 min, Sigma). Into another tube a 10µl aliquot is transferred, to which 1.2µl of 10 × BamHI reaction mixture (100mM Tris-HCl (pH 8 .0), 70mM magnesium chloride, 1000mM sodium chloride, 20mM 2-mercaptoethanol and 0.1% bovine serum albumin) are added. One unit of BamHI is added thereto and the reaction is performed at 37°C for 1 hr. After the reaction, 0.8µl of the mixture is applied to agarose gel electrophoresis.

### (F) Investigation of Structure of pBR322/hag93

### (a) Purification of pBF322/hag93 DNA

From strain W3623Hfla-am76 (pBR322/hag93) pBR322/hag93 cc-DNA is purified in a similar manner to ①-D-a.

### (b) Digestion of pBR322/hag93 cc-DNA with Hinc II

To 50µl of a mixture containing 10mM Tris-HCl (pH 8.0), 7mM magnesium chloride, 60mM sodium chloride, 7mM 2-mercaptoethanol and 1µg of pBR322/hag93 3 units of HincII (0.5µl) are added and the mixture is incubated at 37°C for 30 min. The size of the fragments digested by Hinc II is measured by agarose gel electrophoresis in a similar way to ①-B-a. As a result, fragments of 3.4, 1.85, 1.68, 0.53 and 0.41 kb are observed. Thus, 5 restriction sites of Hinc II on pBF322/hag93 are estimated as shown in Fig.6.

### (c) Subcloning of Hinc II fragment of 1.68 kb into pUC9

It is deduced that the hag gene exists in the fragment of 1.68 kb (①-F-b) between Hinc II restriction sites (5) and (4). This 1.68 kb fragment is subcloned in Hinc II site on plasmid vector pUC9.

In a similar manner to ①-B-a, pBF322/hag93 cc-DNA is digested with EcoRI; 30.5µl of a mixture containing 10µg of pBR322/hag93 cc-DNA and 18 units of EcoRI is incubated at 37°C for 1.5 hr. To 29.5µl of the mixture 5µl of 1M Tris-HCl (pH 8.0) and 63µl of water are added. Further, 0.236 units/2µl of alkaline phosphatase of *E.coli* (BAP, Takara Shuzo Co.,Ltd., Lot 1112) is added thereto and allowed to react at 65°C for 30 min. The mixture is extracted twice with the same volume of phenol and chloroform (1:1) and then twice with the same volume of chloroform following ethanol precipitation. The precipitate is dissolved in 20µl of TE buffer solution. To 20µl of the mixture (10µg DNA) 20 units/4µl of HincII are added. After adjusting the total volume to 40µl, the reaction is performed at 37°C for 2 hr in a similar way to ①-F-b. The reaction is inhibited by heating to 65°C for 10 min. Ethanol precipitation is performed and the precipitate is dissolved in 20µl of TE buffer solution.

Besides, in a similar way to ①-B-a 5µg of plasmid vector pUC9 (Pharmacia)are digested in 30µl of a reaction mixture containing 15 units of Hinc II. The reaction is performed et 37°C for 1.5 hr and then inhibited by heating to 60°C for 10 min. Ethanol precipitation is performed and the precipitate is dissolved in 6µl of TE buffer solution.

DNA linking reaction is performed in a reaction mixture containing 5µg/5µl of pBR322/hag9 digested with EcoRI, BAP and Hinc II, 5µg/6µl of plasmid vector pUC9 digested with Hinc II and 0.1 units/1µl of T4 DNA ligase according to ①-C. With the resultant strain W3623fla-am76 is transformed. Colonies formed on LB medium containing 50 µg/ml of ampicillin are inoculated into the medium for the motility test containing 50 µg/ml of ampicillin. Twelve colonies of 1,112 colonies formed show motility. Two strains thereof are applied to the rapid plasmid DNA analysis. A recombinant plasmid, which is digested to form 1.68 kb fragment with Hinc II and 0.44 and 4.0 kb fragments with BamHI, is named pUC9/hag6 (see Fig.7).

Recombinant plasmid pBR322/hag93 has a HincII fragment of 1.68 kb shown in ①-F-b. The Hinc II fragment gives strain W3623Hfla-am76 motility when the fragment is inserted in plasmid vector pUC9 in right direction (from Hinc II (5) to BamHI** in the fragment) according to the direction of transcription of lac promoter. Thus, the 1.68 kb fragment completely contains a structural gene of the hag gene in the direction from Hinc II (5) to BamHI**.

### (d) Subcloning of Hind III-BamHI fragment of recombinant plasmid pUC9/hag6 in plasmid vector pUC8

From strain W3623Hfla-am76 (pUC9/hag6) the pUC9/hag6 cc-DNA is purified in a similar manner to ①-D-a.

In 160µl of a reaction mixture containing 36 units/6µl of EcoRI, 36 units/3µl of Hind III, 30 units/6µl of Pvu II and 15µg/5.1µl of pUC9/hag6 cc-DNA the pUC9/hag6 cc-DNA is digested with EcoRI, Hind III and Pvu II at 37°C for 2 hr. Besides, in 60 µl of a reaction mixture containing 18 units/3µl of EcoRI, 18 units/1.5µl of Hind III and 5µg/20µl of pUC8(Pharmacia) the pUC8 is digested with EcoRI and Hind III at 37°C for 2 hr according to ①-B-a.

In the above both reactions ethanol precipitaion is performed by extracting twice with phenol and chloroform (1:1) and then twice with chloroform. In the reaction of pUC9/hag6 the precipitate is dissolved in 30µl of TE buffer solution and in the reaction of pUC8, the precipitate is dissolved in 10µl of TE buffer solution. Then, DNA linking reaction is performed in 21µl of a reaction mixture containing 4µg/8µl of digested pUC9/hag6, 2µg/4µl of digested pUC8 and 1 unit/1µl of T4 DNA ligase according to ①-C. After the reaction, strain W3623Hfla-am76 is transformed therewith. Colonies formed on LB agar medium containing 50µg/ml of ampicillin are investigated by the rapid plasmid analysis as to whether or not they have recombinant cc-DNA of 4.4 kb (2.7 kb (pUC8) + 1.68 kb) to give 40 colonies having 4.4 kb cc-DNA. The motility of these 40 colonies is investigated on the medium for the motility test containing 50µg/ml of ampicillin. As a result, none of these 40 colonies shows motility. This means that the hag gene is not expressed when the 1.68 kb fragment is inserted downstream of the lac promoter of pUC8 in reverse direction to that of the lac promoter.

Thus, the 1.68 kb fragment includes the protein coding region of the hag gene (①-F-c) but not its complete promoter region.

A recombinant plasmid carried by one of the above 40 colonies is named pUC8/hag6 and purified in accordance with ①-D-a.

### ② Sequencing of the hag gene

### (A) Sequencing of the hag gene

As it is revealed that the protein coding region of the hag gene is involved in the Hinc II fragment of 1.68 kb (①-F-c and ①-F-d), this fragment is sequenced.

The sequencing of the 1.68 kb fragment is achieved by the dideoxynucleotide chain termination method employing M13 phage vector. Certain fragments (identified by arrows in Fig. 8) produced by digesting cc-DNA of pBR322/hag93, pUC9/hag6 and pUC8/hag6 with Hap II, Hinc II, Taq I, Sau3A, BamHI and Pst I (Takara Shuzo Co.,Ltd.) are cloned in M13 mp8, mp9 and mp18.

The pUC8/hag6 or pUC9/hag6 DNA is denaturated in a solution containing 0.1M sodium hydroxide and 0.1mM EDTA-Na₂ to provide single strand DNA, which is applied to the dideoxynucleotide chain termination method for sequencing of the 1.68 kb fragment.

Fig. 8 shows the scheme for sequencing. The lines in the figure are shown in direction of 5' to 3'.

Fig. 9 shows the whole primary structure of the hag gene determined in the above sequencing. The DNA sequence from the initiation codon ATC downstream to the 60th base of the hag gene is identical with the known DNA sequence (J. Bacteriol. 155, 78 (1983)).

### (B) Determination of amino acid residues at amino and carboxy termini of the flagellin protein

### (a) Purification of flagellin

*E.coli* K-12 W3110 is purified in LB agar medium and incubated overnight at 37°C for colonization. A suitable colony is inoculated into the medium for the motility test and is incubated at 37°C for 4-5 hr. The strain lying around the periphery of the motile zone is transplanted in 100ml of LB broth and is incubated overnight at 37°C. The culture (3-4ml/plate) is spread on LB agar medium in a plate (80 × 225mm, Eiken) and is incubated overnight at 37°C. On the surface of the culture a small amount of buffer solution P (0.15M sodium chloride and 10mM potassium dihydrogenphosphate-disodium hydrogenphosphate, pH 6.8) is poured. The resulting culture is collected with a spreader (Nissui) and suspended in buffer solution P.

From 90 plates about 250ml of the above suspension are prepared and then transferred into 1L-Erlenmeyer flask with cap, shaken hard 600 times and centrifuged at 8,000 r.p.m. at 4°C for 15 min. The supernatant is filtered through glasswool. After centrifuging the filtrate at 25,000 r.p.m. at 4°C for 1 hr (Beckman Ultracentrifuge, rotor #30), the precipitate is suspended in about 20ml of buffer solution P. After centrifugation at 8,000 r.p.m. at 4°C for 15 min, the supernatant, which contains flagellin, is recovered and heated to 65°C for 5 min. After centrifugation at 25,000 r.p.m. at 4°C for 90 min (Beckman Ultracentrifuge, rotor #30), the supernatant (14.5 ml) is recovered to give a crude flagellin fraction.

Then, the crude flagellin fraction is diluted 3 times with 10mM potassium dihydrogenphosphate-disodium hydrogenphosphate solution (pH 6.8) containing 0.05M sodium chloride and applied to a DEAE-cellose column (DE52, Whatman Chemical Separation), which is washed with 98 ml of the above solution for dilution and eluted with 0.05-0.7M sodium chloride dissolved in 10mM potassium dihydrogenphosphate-disodium hydrogenphosphate (pH 6.8) according to the density gradient technique. Flagellin is eluted (18.8 ml) when the concentration of sodium chloride is 0.3M. Employing bovine serum albumin as a standard protein, the flagellin protein is measured by a protein assay kit (Bio-Rad) to be 26.17mg/18.8ml.

### (b) Determination of amino acid residues at amino and carboxy termini and of amino acid composition

Prior to analyzing, purified flagellin prepared in ②-B-a is centrifuged, concentrated by 6.5mg/ml, and then dialyzed to give a starting sample for each analysis.

Determination of amino acid residues of amino terminus is achieved according to the Edman method. As a result the sequence of the N terminal is determined to be Ala(1)-Gln(2)-, which is identical with Ala(1)-Gln(2)- deduced from the DNA sequence shown in Fig. 9.

Determination of the amino acid residue of the carboxy terminus is performed in accordance with the usual method using carboxypeptidase p. As a resuit the amino acid of the C terminal is determined to be Gly, which is identical with Gly(497) deduced from the DNA sequence shown in Fig. 9.

For the analysis of amino acid composition, firstly the purified flagellin (6.5mg/ml) is dissolved in 0.1% ammonia water. To 10µl of the resulting mixture 50µl of 4M methansulfonic acid containing 0.2% indolethylamine, are added and the flagellin in the mixture is hydrolysed at 110°C for 24 hr. To the resultant 47µl of 4N sodium hydroxide and 403µl of 0.2N sodium citrate are added to prepare a sample for the analysis. The analysis is achieved with Hitachi amino acid analyzer model 835. Table 1 shows a comparison of the found amino acid composition with that deduced from the DNA sequence.

**Table 1**

| Amino Acid Composition of Flagellin of *E*. *coli* K-12 | | |
|---|---|---|
| Amino acid | Found(a) | Calcd. |
| Ala | 58.1 | 59 |
| Val | 32.8 | 33 |
| Leu | 37.5 | 37 |
| IIe | 27.0 | 28 |
| Gly | 45.3 | 44 |
| Pro | 6.0 | 6 |
| Cys | 0.0 | 0 |
| Met | 2.7 | 3 |
| His | 0.0 | 0 |
| Phe | 5.0 | 5 |
| Tyr | 9.9 | 10 |
| Trp | 0.0 | 0 |
| Asn(+Asp) | (88.7) | 48(87) |
| Gln(+Glu) | (41.5) | 27(41) |
| Ser | 42.8(b) | 43 |
| Thr | 63.3(b) | 65 |
| Lys | 25.4 | 25 |
| Arg | 10.2 | 11 |

| | | |
|---|---|---|
| a. Calculated by regarding Phe residue as 5.0. | | |
| b. Revised by taking hydrolysis into consideration. | | |

### ③ In vitro preparation of bag gene variant in H antigencity

### (A) Digestion of pBR322/hag93 ccDNA with deoxyribonuclease I (DNase I)

pBR322/hag93 ccDNA (1.35mg, 1ml) is subjected to ethanol precipitation in TE buffer solution and then dissolved in 50ml Tris-HCl (pH7.3). A reaction mixture (340µl) containing 125µg of the DNA, 6ng of DNase I (Takara Shuzo Co.,Ltd.) and 1mM manganese chloride in 50mM Tris- HCl (pH7 .3) is incubated at 37°C for 10 min and then immediately the same volume of phenol-chloroform (1:1) is added thereto for inhibiting the reaction. The resultant is applied to phenol-chloroform extraction in the way similar to ①-E. After ethanol precipitation, the precipitate is dissolved in 200µl of TE buffer solution.

The solution is separated by agarose gel electrophoresis and DNA is extracted from the gel piece of the fluorescent band corresponding to linear monomer (as size marker of linear monomer pBR322/hag93 DNA digested with EcoRI is employed). The extract is subjected to ethanol precipitation to give about 2µg of linear pBR322/hag93 DNA.

### (B) Digestion with exonuclease Bal31

The DNA in ③-A is dissolved in 14µl of water and distributed into 7 fractions. Than, to 2µl of aliquots, 2µl of Bal31 reaction mixture (24mM calcium chloride, 24mM magnesium chloride, 200mM sodium chlorlde, 40mM Tris-HCl (pH8.0) and 2mM EDTA-Na₂) are added and the mixture is incubated at 30°C for 3min.

Bal31 enzyme solution (Takara Shuzo Co.,Ltd.) is diluted in a solution comprising 12mM calcium chloride, 12mM magnesium chloride, 100mM sodium chloride, 20mM Tris-HCl (pH8.0), 1mM EDTA-Na₂ and 0.1% bovine serum albumin to 6.02, 3.01, 1.51, 0.76, 0.38, 0.19, 0.10 unites/ml. To the ahove DNA mixture 4µl of the BAl31 mixture of each concentration are added and the reaction mixture is incubated at 30°C for 10 min. Then 1µl of ethylene glycol bis(β-aminoethyl ether)-N, N, N', N'-tetraacetic acid is added thereto and the mixture is ice-chilled. The contents of the 7 reaction tubes are put together and applied to phenol chloroform extraction and ethanol precipitation in a way similar to ①-E.

### (C) Repair with E. coli DNA polymerase I large fragment (klenow fragment) and ligation in the presence of Hind III linker

The DNA of ③-B is dissolved in 3µl of TE buffer solution. This 3µl of the DNA (about 2µg) is incubated at 30°C for 30 min with 1µl of 10×nick translation buffer (0.5M Tris-HCl (pH7.2), 0.1M magnesium sulfate, 1mM dithiothreitol and 500µg/ml bovine serum albumin), 4µl of dNTPs (1mM dATP, 1mM dTTP, 1mM dGTP and 1mM dCTP, Takara Shuzo Co., Ltd.). 1µl of klenow fragment (3 units/µl, Takara Shuzo Co., Ltd.) and 1µl of water. Then, a solution comprising 10µl of the above reaction mixture, 4µl of 5×ligation buffer (0.25M Tris-HCl (pH7.4), 0.05M magnesium chloride, 5mM spermidine and 0.5mg/ml bovine serum albumin), 1µg/1µl of Hind III linker (5'-CAAGCTTG, Takara Shuzo Co., Ltd.). 2µl of 100mM dithiothreitol, 2µl of 10mM ATP and 2 units/2µl of T4DNA ligase is incubated at 16°C for 16 hr

### (D) Transformation and selection of the strain variant in H-antigencity

*E. coli* K-12 C600 r⁻m⁻ hag::Tn10 is transformed with the above resultant in ③-C. The strain C600r⁻m⁻ hag::Tn10 is a r⁻ (restriction minus) m⁻ (modification minus) descendent from the strain C600 (thr-1, leu-6, thi-1, supE44, lacY1, tonA21, λ⁻) and has an inactive hag gene in which Tn10 in inserted. About 8,600 colonies of transformants are formed on LB agar medium containing 50µg/ml of ampicillin. About 5,000, colonies thereof are incubated at 37°C for 5-6 hr on the medium for the motility test containing 1/10⁴ volume of anti-flagella polymer antiserum. Twelve strains (group A) which show good motility as well as that on the medium for the motility test containing no antiserum are selected. Further, 4 strains (group B) which show apparent motility in incubation at 37°C for 20 hr even in the presence of the antibody are selected.

An antigen used in preparing anti-flagella polymer antiserum is prepared by the method as follows. To 13.9 mg/10 ml of purified flagellin ammonium sulfate by the concentration of 60% (w/v) is added and the mixture is incubated overnight at 4°C and then centrifuged at 15,000 g for 15 min. The precipitate is suspended in the buffer solution P (1 .3 ml) and dialyzed against the buffer solution P. As an adjuvant an incomplete adjuvant (DIFCO) is employed. At first 3 mg of the antigen is subcutaneously injected to each rabbit (New Zealand White) at both foot pads and the thigh. On days 9 and 14 the same treatments are performed. On day 21 the whole blood is drawn from the carotid artery. Thereafter a crude serum fraction is prepared by the usual method.

### (E) Analysis of the hag gene of the strain variant in H-antigencity

### (a) Rapid plasmid DNA analysis

Whether the plasmids carried by the groups A and B selected in ③-D are digested with Hind III or not is analysed by the rapid plasmid DNA analysis method.

The plasmids of 4 strains from the group A and 1 strain from the group B are digested with Hind III. Since these plasmids are originally derived from the plasmid pBR322/hag93 which does not have a HindIII-cutting site, the HindIII-cutting site in these plasmids must be generated by the insertion of the synthesized HindIII linker (③-C).

### (b) Location of Hind III restriction site on the plasmid

Plasmids of 3 strains of the 4 strains from the group A in ④-E-a are named pFD1, pFD2 and pFD3, respectively. The plasmid carried by one strain of the group B is named pHD-1. The location of the HindIII restriction site on each of these 4 plasmids is determined by the rapid plasmid DNA analysis method.

The plasmids pFD1, pFD2 and pFD3 prepared by the rapid plasmid DNA analysis method are digested with EcoRI and HindIII and the sizes of the digested fragments are measured by the agarose gel electrophoresis.

Fragments of about 5.3 kbp and about 2.3 kbp are observed in all the samples. Accordingly, it is concluded that the HindIII restriction site lies at the position about 2.3 kbp apart from EcoRI restriction site (①-F-b). While the pBR322/hag93 DNA was digested with BamHI into fragments of about 5.8 kbp and about 1.9 kbp (①-C), the BamHI fragment of 1.9 kbp of pFD1, pFD2 and pFD3 is cut into 2 fragments by digestion with Hind III. This means that the Hind III restriction site of each sample is about 2.3 kbp apart from the EcoRI restriction site in the clockwise direction and lies in the BamHI fragment of about 1.9 kbp. The distance from the EcoRI restriction site to the first BamHI restriction site in the clockwise direction is about 0.7 kbp (①-C). And the Hinc II restriction site (①-F-b), which is located approximately 70 bp upstream from the initiation codon of the hag gene, is about 0.7kbp distant*¹ from the first BamHI restriction site in the clockwise direction. Accordingly, it is concluded that the EcoRI restriction site is 1.4 kbp distant from the DNA region of the hag gene in the clockwise direction and that the Hind III restriction site about 2.3 kbp apart from the EcoRI restriction site in the clockwise direction lies in the DNA region of the hag gene (see Fig. 10).
*1 In ①-F-b and Fig. 6 the distance from Hinc II (1) to Hinc II (5) is 1.85 kbp and the distance from EcoRI to BamHI* is about 0.73 kbp. Besides, the distance from Hinc II (1) to EcoRI is about 0.45 kbp (J.G. Sutcliffe, 1979, Cold Spring Harbor Lab. Symposia 43, p. 83). Accordingly, the distance from BamHI* to Hinc II (5) is about 0.7 kbp.

In the way similar to the above method, the location of the Hind III restriction site on the plasmid pHD-1 is determined. The plasmid pHD-1 is digested with EcoRI and HindIII into fragments of about 6.4 kbp and about 1.4 kbp. Accordingly, the HindIII site is clarified to be about 1.4 kbp distant from the EcoRI site. Besides, it is digested with BamHI and HindIII into fragments of about 5.8 kbp, about 1.2 kbp and about 0.7 kbp. This means that on the plasmid pHD-1 the Hind III site is 1.4 kbp distant from the EcoRI site in the clockwise direction and lies at around Hinc II (5) site in Fig. 10, which is also around the initiation codon ATG of the hag gene.

### (C) Analysis of DNA lacking region in plasmid pFD1, pFD2 and pFD3.

In the step of the in vitro preparation of plasmid pFD1, pFD2 and pFD3 the site cut by DNase I is somewhat digested with Ba131 (③-B). The deficiency of DNA by this digestion is deduced from the position where the synthesized HindIII DNA linker is inserted. This position of the insertion is about 2.3 kbp distant from the EcoRI site on the plasmid in the clockwise direction and lies in the Hinc II (5) - Nde1*¹ fragment.
*1 The restriction enzyme Nde1 restricts a base sequence 5'CA'TATG, which lies at 953th nucleotide in Fig. 9 and at about 2.4 kbp downstream from the EcoRI site and at about 1kbp downstream from the Hinc II (5) site, respectively.

DNAs of pFD1, pFD2, pFD3, and pBR322/hag93 obtained by the rapid method are digested with Hinc II and Ndel and the fragments are analysed by the agarose gel electrophoresis.

From Fig. 9 it is deduced that the Hinc II fragment having the hag gene has a length of 1.7kbp and falls after digestion with Ndel into about 1.0 kbp and 0.7 kbp fragments. In the experiment the Hinc II (5)-Ndel fragments from pBR322/hag93, pFD1, pFD2 and pFD3 are 1,020bp, 930bp, 990bp and 990bp in length, respectively. Accordingly, it is revealed that the Hinc II (5)-Ndel fragments, of pFD1, pFD2 and pFD3, which have a HindIII site, are about 90bp. about 30 bp and about 30 bp shorter than that of pBR322/hag93, respectively.

### (d) DNA sequencing at around Hind III restriction site on pFD1, pFD2 and pFD3.

The plasmids pFD1, pFD2 and pFD3 are purified by using the host C600r⁻m⁻hag::Tn10 in the way similar to ①-D-a. DNA sequencing of the purified plasmids at around Hind III site (Fig. 11) is performed by the usual dideoxynucleotide chain termination method using M13 phage. M13mp18 (Pharmacia) is digested with Hind III and BamHI, extracted with phenol chloroform, then ethanol precipitated and finally dissolved in TE buffer solution. The digested DNA is ligated with T4DNA ligase and introduced into strain JM105 (Pharmacia) to form plaques. According to the usual method, the recombinant M13mp18 DNA having fragment A or B in Fig.11 is prepared. After cloning in the M13mp18 vector as noted above a single strand DNA is prepared and sequenced by the dideoxynucleotide chain termination method.

The hag gene carried on pFD1 lacks the sequence from bp 754 to 854 in Fig. 9, into which the synthesized HindIII linker 5'CAAGCTTG is inserted. As a result an amino acid sequence NDGTVTMATGATANATUTDANTTKATTITSGGTP(34 amino acid residues) is deleted end QAC is inserted. The hag gene carried on pFD2 lacks the sequence from bp 719 to 765 in Fig.9, into which the synthesized Hind III linker 5'CAAGCTTG is inserted. As a result an amino acid sequence DNDGKYYAVTVANDGT (16 amino acid residues) is deleted and ASL is inserted. The hag gene carried on pFD3 lacks the sequence from bp 778 to 836 in Fig. 9, into which the synthesized HindIII linker 5'CAAGCTTG is inserted. As a result an amino acid sequence TGATANATVTDANTTKATTI (20 amino acid residues) is deleted and QAC is inserted.

### (e) DNA sequencing at around Hind III restriction site on plasmid pHD1.

The cc-DNA of pHD1 is purified from the strain C600 r⁻m⁻ bag::Tn10(pHD1) in the way similar to ①-D-a. The DNA sequencing of the purified plasmid DNA at around Hind III (Fig. 12) is performed by the dideoxynucleotide chain termination method using M13mp18 phage vector as noted in ③-E-d by preparing a single strand DNA of the recombinant M13mp18 having fragment A or B in Fig. 12. As a result, the bag gene carried on pHD1 lacks 8 bases 5'AACGACTT of No. -28 to -21 in Fig. 9, into which 8 bases 5'CAAGCTTG (synthesized Hind III linker) are inserted. This mutation is named pHD1 mutation. It is revealed from the DNA sequencing that the pHD1 mutation did not occur in the region encoding protein in the bag gene. Accordingly, the flagellin coded in the plasmid pHD1 is the same as natural flagellin and so the H antigencity of the constructed flagella also does not alter.

### ④ The number of flagella of the host strain C600r⁻m⁻ hag::Tn10 having the plasmid pHD1.

Flagella of C600r⁻m⁻hag::Tn10 having pHD1 and flagella of C600r⁻m⁻hag::Tn10 having pBR322/hag93, which are incubated overnight at 30°C, are dyed. The number of the bacteria having the same number of flagella is counted under an optical microscope (Table 2).

**Table 2**

| The number of bacteria having the same number of flagella | | |
|---|---|---|
| Plasmid | pBR322/hag93 | pHD1 |
| Number of flagella | | |
| 0 | 249 | 109 |
| 1 | 37 | 79 |
| 2 | 10 | 46 |
| 3 | 2 | 38 |
| 4 | 1 | 15 |
| 5 | 1 | 9 |
| 6 | 0 | 2 |
| 7 | 0 | 1 |
| 8 | 0 | 1 |
| host: C600r⁻m⁻hag::Tn10 | | |

The strain C600r⁻m⁻hag::Tn10 having the plasmid pHD1 forms the more number of flagella. When the motility is compared between the both strains on the medium for the motility test in the manner similar to ①-A-a, the averaged of diameters of motile zones after incubation at 37°C for 5 hr are 16mm in pBR322/hag93 and 36mm in pHD1, which reveals the strain carrying pHD1 has a better motility. A multiple copy plasmid such as pBR322 having the hag gene with the pHD1 mutation makes the host E.coli to form a larger number of flagella than that plasmid having the natural hag gene.

### ⑤ Construction of pFD202

### (A) Digestion of pFD2 with nuclease Bal31

A cc-DNA of pFD2 is purified from the strain C600r⁻m⁻hag::Tn 10 (pFD2) in a similar way to ①-D-a. To a mixture of 20µg/6.2µl of pFD2 ccDNA, 10µl of 10 x Pvu II buffer solution (100mM-Tris-HCl (pH7.5), 70mM magnesium chloride, 600mM sodium chloride and 70mM 2-mercaptoethanol) and 73.8 µl of water 120 units/10µl of HindIII are added and the reaction mixture is incubated at 37°C for 1 hr. The resultant is extracted with phenol chloroform, ethanol precipitated and finally dissolved in 20µl of 0.05% bovine serum albumin solution. To 4µl of the resulting DNA solution 4µl of 2 x Bal31 buffer solution (③-B) are added and the reaction mixture is incubated at 30°C for 3 min. Then, 8µl of nuclease Bal31 (4,900 units/ml) diluted 520 times with Bal31 buffer solution are added thereto and the reaction mixture is incubated at surrounding temperature for 10 min, and after adding of 2 µl of 200mM EGTA (③-B) thereto the mixture is ice-chilled. After phenol chloroform extraction and ethanol precipitation the precipitate is dissolved in 10 µl of TE buffer solution.

### (B) Repair with klenow fragment and ligation in the presence of synthesized DNA linker (Hind III-Sma I -Bgl II)

To 5µl of the sample DNA (about 4µl/10µl TE buffer solution) in ⑤-A 1µl of 10 x nick translation buffer, 4µl of dNTP's (③-C) and 3 units/1µl of klenow fragment are added and the reaction mixture is incubated at room temperature for 30 min. Then, the above reaction mixture is mixed with 5 x ligation buffer (③-C) 0.5µg/1µl of synthesized DNA liner (Hind III - Sma I -Bgl II, double strands 18 mer polynucleotide 5'AAGCTTCCCGGGAGATCT3' and 3'TTCGAAGGGCCCTCTAGA5'; chemically synthesized according to the solid phase synthesis employing triphoshate), 2µl of 10mM ATP, 2µl of 100mM DTT and 2µl of water. To the resulting mixture 2 units/2µl of T4DNA ligase are added and the reaction mixture is incubated at 14°C for 18 hr.

### (C) Selection of transformants

*E. coli* K-12 C600r⁻m⁻hag::Tn10 is transformed with the reaction mixture in ⑤-B. About 1,200 colonies are formed on LB agar medium containing 50 µl/ml ampicillin. These colonies of transformants are inoculated with a sterilized toothpick into the medium for the motility test containing 50 µg/ml ampicillin and incubated at 37°C for 5-6 hr. About 50% of the colonies show motility and 90 strains thereof are investigated whether their plasmids are digested with Sma I by using the rapid plasmid DNA analysis method. As a result, plasmids carried by 2 strains are digested with Sma I. A plasmid carried by 1 strain thereof is named pFD202, and is also digested with HindIII and BglII.

### (D) DNA sequencing of pFD202 at around the insertion region of synthesized linker

The pFD2 is a little digested with nuclease Bal31 from the unique HindIII restriction site and the resultant plasmid is named pFD202.
Accordingly, the site of the synthesized DNA liner insertion lies at around Hind III site of pFD2 and is included in the about 900 bp fragment generated by digestion of pFD202 with Hinc II and Pst I.

### (a) Digestion of pFD202 with Hinc II and Pst I

A cc-DNA of pFD202 is purified from the strain C600r⁻m⁻ hag::Tn10 (pFD202) in a manner similar to ①-D-a. The pFD202 cc-DNA (23µg/10µl) is mixed with 10µl of 10 x Pvu II buffer solution, 10µl of 70mM 2-mercaptoethanol, 5µl of 0.2% bovine serum albumin, 60 units/10µl of Hinc II and 96 units/6µl of Pst I. After the volume is adjusted to 100µl, the mixture is incubated at 37°C for 80 min. After the reaction 10µl of 5 x sample buffer (25% sucrose, 5mM sodium acetate, 0.1% sodium dodecyl sulfate and 0.05% brome phenol blue) are added thereto. Its whole volume is applied to electrophoresis of 0.8% agarose gel at 100V for 40 min. After staining with 0.5µg/ml ethidium bromide, the gel piece containing the about 900 bp band is cut off. After twice electric extraction at 10V for 10 min, the resultant is ethanol precipitated and then dissolved in 200 µl of TE buffer solution.

### (b) Digestion of M13mp8 DNA with Hinc II and Pst I

To a reaction mixture containing 3 µg/30µl of M13mp8 RFDNA, 10µl of 10 x Pvu II buffer solution, 5µl of 70mM 2-mercaptoethanol and 40µl of water 18 units/3µl of HincII and 32 units/2µl of Pst I are added and the reaction mixture is incubated at 37°C for 1 hr. After phenol chloroform extraction and ethanol precipitation, the precipitate is dissolved in 21µl of TE buffer solution.

### (c) Cloning of Hinc II -Pst I fragment in vector M13mp8 and its DNA sequencing

To 21 µl of reaction mixture containing 10µl of TE buffer solution containing Hinc II -Pst I fragment of ⑤-D-a, 3µl (about 430 ng DNA) of the sample of ⑤-D-b, 4µl of 5 x ligation buffer, 2µl of 100mM DTT and 2µl of 10mM ATP 1 unit/1µl of T4DNA ligase is added and the reaction mixture is incubated at 14°C for 18 hr. A strain JM109 (Takara Shuzo Co., Ltd.) is transformed with the whole volume of the resulting mixture.

After cloning in a vector M13mp8, the single strand DNA is prepared and sequenced in accordance with the dideoxynucleotide chain termination method. As a result, the hag gene carried on the plasmid pFD202 lacks 78 base pairs (711th to 788th in Fig.9) from the wild hag gene, but in place of these sequences 18 other base pairs are inserted. Namely, natural 26 amino acid resides are lacked and 6 artificial amino acid residues are inserted therein in terms of peptide.

### ⑥ Construction of plasmids pFD301, pFD303, pFD306 and pFD307

### (A) Digestion of pFD3 with nuclease Bal31

A cc-DNA of pFD3 is purified from the strain C600r⁻m⁻ hag::Tn10 (pFD3) in a way similar to ①-D-a. To a mixture of 50µg/12µl of pFD3 ccDNA, 25µl of 10 x Pvu II buffer solution and 188 µl of water 300 units/25µl of HindIII are added and the reaction mixture is incubated at 37°C for 1 hr. After the reaction mixture is applied to phenol chloroform extraction and ethanol precipitation in a manner similar to ①-E the precipitate is dissolved in 25µl of water. To 5 µl (about 2µl DNA/µl) of the resulting mixture 12.5 µl of 0.2% bovine serum albumin solution and 32.5µl of water are added. The final DNA conc. is about 200ng/µl in 0.05% bovine serum albumin. To 20 µl of the solution 20 µl of 2 x Bal31 buffer solution are added and the reaction mixture is incubated at 30°C for 30 min. A solution (40µl) prepared by diluting nuclease Bal31 (1,700 units/ml) 1,140 times with Bal31 diluter is added thereto. After reaction at room temperature for 20 min, 5µl of 100mM EGTA are added to 20µl of the resulting mixture and this mixture is then ice-chilled. After phenol chloroform extraction and ethanol precipitation, the precipitate is dissolved in 10µl of water.

### (B) Repair with klenow fragment and ligation in the precense of synthesized DNA linker (HindIII-SmaI-BalII)

To the DNA sample (about 1µg/10µl) of ⑥-A 1.5µl of 10 x nick translation buffer, 2µl of dNTPs and 3 units/1µl of klenow fragment are added and the reaction mixture is incubated at room temperature for 30 min. Then, to the resulting mixture 3.7µg/5µl of synthesized DNA linker (HindIII-SmaI-BglII), 7µl of 100mM DTT, 3 µl of 10mM ATP, 1µl of 500mM Tris-HCl (pH 7.3), 1.5µl of water and 2 units/2µl of T4DNA ligase are added and the reaction mixture is incubated at 15°C for 18hr.

### (C) Selection of transformants

After dialyzing the resulting mixture of ⑥-B against TE buffer solution for about 4 hr, *E. coli* K-12 C600r⁻m⁻hag::Tn10 is transformed with the resultant. About 560 colonies of transformants are formed on LB agar medium containing 50µg/ml of ampicillin. These colonies are inoculated with a sterilized toothpick into the medium for the motility test containing 50µg/ml of ampicillin. Including strains showing weak motility, 23 motile strains are picked up. It is investigated on these 23 strains whether plasmids carried by them are digested with Sma I and as a result 4 strains are selected. Plasmids carried by the 4 strains are named pFD301, pFD303, pFD306 and pFD307. These 4 plasmids are also digested with HindIII and BglII, shown by the rapid plasmid DNA analysis.

### (D) DNA sequencing of pFD301, pFD303, pFD306 and pFD307 at around an insertion region of synthesized DNA linker.

The plasmids pFD301, pFD303, pFD306 and pFD307 derives from FD3 by the digestion with nuclease Bal31 from Hind III restriction site. Therefore, an insertion region of synthesized DNA linker on pFD301, pFD303, pFD306 and pFD307 lies at around Hind III restriction site of pFD3. Accordingly, DNA sequencing at around the insertion region of synthesized DNA linker is achieved from the HindIII site of the 2 HindIII-HincII fragments A' and B' (see Fig. 13).

### (a) Digestion of pFD301, PFD303, pFD306 and pFD307 with Hinc II and Hind III

A cc-DNA is purified from the strains C600r⁻m⁻hag::Tn10 having the plasmids pFD301, pFD303, pFD306 and pFD307 according to ①-D-a. To a mixture of 5µg of pFD301 ccDNA, 5µl of 10 x Pvu II buffer solution, 5µl of 70mM 2-mercaptoethanol, 30 units/5µl of Hinc II and 50 units/5µl of Hind III water is added for adjusting the volume to 50µl and the mixture is incubated at 37°C for 1hr. After phenol chloroform extraction and ethanol precipitation, the precipitate is dissolved in 10µl of water. The same treatment is performed on pFD303, pFD306 and pFD307.

### (b) Digestion of M13mp18 RFDNA with Hinc II and Hind III

To a reaction mixture containing 2.5µg/5µl of M13mp18 RFDNA, 5µl of 10 x Pvu II buffer solution, 5µl of 70mM 2-mercaptoethanol and 25µl of water, 30 units/5µl of HincII and 50 units/5µl of HindIII are added and the reaction mixture is incubated at 37°C for 12hr. After phenol chloroform extraction and ethanol precipitation, the precipitate is dissolved in 10µl of water.

### (c) Cloning of Hinc II -Hind III fragment in vector M13mp18 and its DNA sequencing

To a mixture containing 1.5µg/3µl of Hinc II-Hind III fragment in ⑥-D-a, 0.5µg/2µl of the sample in ⑥-D-b, 4µl of 5 x ligation buffer. 2µl of 100mM DTT, 2µl of 10mM ATP and 5µl of water, 2 units/2µl of T4DNA ligase are added and the reaction mixture is incubated at 15°C for 18hr. The strain JM109 (Takara Shuzo Co., Ltd.) is transformed with the whole volume of the resulting mixture. Picking up 10 plaques, a single strand DNA is prepared in accordance with the usual method. A double strand DNA digested with Hinc II and Hind III is investigated by 1.6% agarose gel electrophoresis (pFD3 cc-DNA digested with Hinc II and Hind III is used as a control of size of DNA fragment). As a result both of the fragments A and B derived from pFD3 ccDNA are disappeared but in place of them fragments A' and B' shorter than fragments A and B are observed. Each sample of the single strand DNA having the above both fragments is applied to DNA sequencing according to the dideoxynucleotide chain termination method. As a result the hag gene carried on pFD301 lacks 561 base pairs (583th to 1,149th), compared with the wild hag gene and in place of those sequences 18 other base pairs are inserted. Namely, natural 187 amino acid resides are lacked from the wild type flagella and 6 artificial amino acid residues are inserted to the pFD301-coded flagellin (Fig. 14-A). The sequences of hag genes of pFD303, pFD306 and pFD 307 are shown in Figs. 14-B, C, and D, respectively.

### ⑦ Construction of pFD311, pFD313 and pFD315

The plasmids pFD311, pFD313 and pFD315 are constructed in the same way as ⑥-A, ⑥-B, ⑥-C and ⑥-D except that the time of the reaction of nuclease Bal31 is 5 min. The DNA sequences of the plasmids pFD311, pFD313 and pFD315 are shown in Fig. 15-A, B and C, respectively.

### ⑧ Excretion of flagellin fused with polypeptide HBsAG- I by plasmid vector pFD1

### (A) Digestion of pFD1 with Hind III and repair with klenow fragment

To a mixture containing 20µg of pFD1 cc-DNA, 10mM Tris-HCl (pH7.5), 7mM magnesium chloride and 60mM sodium chloride 100 units of HindIII are added and the reaction mixture is incubated at 37°C for 30 min. After phenol chloroform extraction and ethanol precipitation, the precipitate is dissolved in 20µl of TE buffer solution.

To the above DNA (20µg/20µl TE buffer solution) 2µl of 10 x nick translation buffer, 2µl of dNTP (1mM of each XTP) and 3 units/1µl of klenow fragment are added and the reaction mixture is incubated at room temperature for 30 min. Then, 1µl of 500mM EDTA is added thereto. After phenol chloroform extraction and ethanol precipitation, the precipitate is dissolved in 20µl of TE buffer solution.

### (B) Annealing of synthesized polynucleotide

To the synthesized polynucleotide RTH-1603 (5'ACAAAACCTACGGATGGAAATGG3', about 20µg/100µl of water) and the synthesized RTH-1604 (5'CCATTTCCATCCGTAGGTTTTGT3', about 20µg/100µl water) 20µl of 10 x TE buffer solution are added. Annealing is achieved by the incubation of the mixture at 60°C for 30 min and by gradually cooling it to 28.5°C in 6 hr.

### (C) Ligation of synthesized DNA with pFD1

A mixture containing 1µg/1µl of the DNA of ⑧-A, 180pmol/13µl of the synthesized DNA of ⑧-B, 4µl of 5 x ligation buffer and 5.6 units/2µl of T4DNA ligase is incubated at 16°C for 16 hr and the resulting mixture is dialyzed against TE buffer solution for 2 hr.

### (D) Selection of transformants

A strain C600r⁻m⁻hag::Tn10 is transformed with the DNA of ⑧-C. On LB medium containing 50µg/ml ampicillin 3,8 X 10⁴ ampicillin-resistant colonies are formed per 1ug of pFD1 DNA. Motility of 90 colonies thereof is investigated and 82 strains show motility. The rapid plasmid analysis is achieved on 62 strains thereof and as a result 5 strains #11, #12, #14, #16 and #17 have a plasmid that is not digested with Hind III. The plasmids carried by these 5 strains are named pFD1/HBsAg- I #11, #12, #14, #16 and #17.

### (E) DNA sequencing at around synthesized DNA inserted into pFD1/HBsAg- I

A region into which the synthesized DNA fragment is inserted on pFD1/HBsAg- I is included in the 3rd largest fragment among the fragments generated by digestion with Hinc II and Pst I.

### (a) Digestion of pFD1/HBsAg- I with Hinc II and Pst I

Each of the 5 strains #11, #12, #14, #16 and #17 of ⑧-D is incubated in 10 ml of LB broth containing 50 µg/ml of ampicillin. For the rapid plasmid DNA analysis a sample of each strain is prepared and dissolved in 150µl of TE buffer solution.

To the above sample 22µl of 10 x PvuII buffer solution, 22µl of 70mM 2-mercapto-ethanol, 11µl of 0.2% bovine serum albumin, 80 units/10µl of HincII and 90 units/12µl of PstI are added and the reaction mixture is incubated at 37°C for 1 hr.

The resulting mixture is concentrated to 10 µl with a centrifuge evaporator to which 100µl of sample buffer were added. The resultant is subjected to 0.8% agarose gel electrophoresis and the gel piece including the 3rd largest fluorescent band is cut off and applied to electric extraction and ethanol precipitation. The precipitate is dissolved in 15µl of TE buffer solution.

### (b) Digestion of M13mp18 RFDNA with Hinc II and Pst I

M13mp18 RFDNA is digested with Hinc II and Pst I according to ⑤-D-b except that 2µg/4µl of M13mp18 RFDNA in place of M13mp8 RFDNA and 66µl of water are used.

### (c) Cloning of Hinc II -Pst I fragment in M13mp18 and DNA sequencing

To 4µl of each DNA sample of strains #11, #12, #14, #16 and #17 of ⑧-E-a 4µl (400ng) of DNA sample of ⑧-E-b, 4µl of 5 x ligation buffer, 2µl of 100mM DTT, 2µl of 10mM ATP, 1µl of water and 1 unit/1µl of T4DNA ligase are added and the reaction mixture is incubated at 16°C for 16 hr. A strain JM109 is transformed with whole volume of the resultant. After cloning in vector M13mp18, a single strand DNA is prepared and applied to the dideoxynucleotide chain termination method for DNA sequencing. As a result a sequence at around synthesized DNA in each plasmid is clarified as follows.
pFD1/HBsAg-I #11 
AlaGluAlaThrLysProThrAspGlyAsnGlyAlaCysVal

5' GCTCAAGCTACAAAACCTACGGATGGAAATGGAGCTTGTGTT
pFD1/HBsAg- I #12,#14, #16, #17 
AlaGlnAlaProPheProSerValGlyPheValAlaCysVal

5'GCTCAAGCTCCATTTCCATCCGTAGGTTTTGTAGCTTGTGTT
Underlined is a synthesized DNA inserted.

### ⑨ Excretion of flagellin fused with polypeptide HBsAg- II by plasmid vector pFD1

### (A) Digestion of pFD1 cc-DNA with Hind III and repair with klenow fragment

A pFD1 cc-DNA is digested with Hind III and repaired with klenow fragment according to ⑧-A

### (B) Annealing of synthesized polynucleotide

Synthesized RSM-2103 (5'ACAAACCTTCGGATGGAAATGG3') AND RSM-2104 (5'CCATTTCCATCCGAAGGTTTTGT3') are annealed in a way similar to ⑧-B.

### (C) Ligation of synthesized DNA with vector pFD1.

The synthesized DNA is ligated with vector pFD1 in accordance with ⑧-C.

### (D) Selection of transformants

A strain C600r⁻m⁻hag::Tn10 is transformed with the sample DNA in ⑨-C. On LB medium containing 50µg/ml of ampicillin 2 x 10⁴ ampicillin-resistant colonies are formed per 1µg of pFD1 DNA. Motility of 68 colonies thereof is investigated and 43 strains thereof show motility. Plasmids carried by 16 strains thereof are applied to the rapid plasmid analysis in accordance with ⑧-D. As a result it is revealed that the plasmids carried by the 7 strains #1,#2, #3, #6, #8, #13 and #16 are not digested with Hind III. The plasmids carried by them are named pFD1/HBsAg- II #1, #2, #3, #6, #8, #3 and #16.

### (E) Sequencing at around synthesized DNA inserted into pFD1/HBsAg- II

DNA sequencing is achieved on pFD1/HBsAg- II #1 and pFD1/HBsAg- II #13 according to ⑧-E. The sequence at around the synthesized DNA on both plasmids is shown below.
pFD1/HBsAg II #1, pFD1/HBsAg II #13, pFD1/HBsAg- II #1, pFD1/HBsAg II #13 
AlaGluAlaThrLysProSerAspGlyAsnGlyAlaCysVal

5'GCTCAAGCTACAAAACCTTCGGATGGAAATGGAGCTTGTGTT
Underlined is a synthesized DNA.

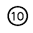 Increase of the number of flagella by pHD1 mutation

### (A) Preparation of cc-DNAs of plasmids pFD1, pFD1/HBsAg- I and pFD1/HBsAg- II from dam⁻ strain

For the rapid plasmid analysis a DNA sample is prepared from the strain C600r⁻m⁻hag::Tn10 having pFD1, pHD1, pFD1/HBs Ag-I #11, pFD1/HBsAg-II#1. A strain GM33 dam⁻ is transformed with each of the above samples to establish GM33dam⁻ (pFD1), GM33dam⁻ (pHD1), GM33dam⁻ (pFD1/HBsAg- I #11) and GM33dam⁻ (pFD1/HBsAg- II #1). A cc-DNA is purified from each strain according to ①-D-a.

### (B) Preparation of EcoRI-Bcl I fragment (about 1.4 kbp) from plasmid pHD1

To 29.7 µg/25µl of pHD1 cc-DNA of 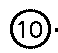 -A 20 µl of 10 x BclI buffer solution (500mM Tris-HCl (pH 8.0 ), 100 mM magnesium chloride and 500mM sodium chloride), 20 µl of 70mM 2-mercaptoethanol, 10µl of 0.2% bovine serum albumin, 75 units/10µl of EcoRI and 105µl of water are added and the reaction mixture is incubated at 37°C for 1 hr. Then, 50 units/5µl of BclI are added thereto and the reaction mixture is incubated at 50°C for 1 hr. A mixture of 189 µl of the resultant and 20µl of sample buffer is applied to 0.8% agarose gel electrophoresis. A fluorescent band corresponding to approximately 1.4 kbp is cut off, applied to electric extraction and dissolved in 30µl of TE buffer solution.

### (C) Digestion with EcoRI, treatment with alkaline phosphatase (BAP) and partial digestion with Bcl I on pFD1, pFD1/HBsAg- I #11 and pFD1/HBsAg- II #1

To 40µg/40µl of pFD1, pFD1/HBsAg-I #1 and pFD1/HBsAg-II #1 16µl of 10 x EcoRI buffer solution (1M Tris-HCℓ (pH 7.5), 70mM- magnesium chloride and 500mM sodium chloride), 16µl of 70mM 2-mercaptoethanol, 8µl of 0.2% bovine serum albumin and 60 units/8µl of EcoRI are added and the reaction mixture is incubated at 37°C for 30min. After adjusting the volume to 160µl by adding water, the resultant is incubated at 37°C for 1hr. Then, to each sample 6µl of 1M Tris-HCℓ (pH 8.0), 1.2 units/10µl of BAP and 25µl of water are added and the reaction mixture is incubated at 65°C For 30 min.

After phenol chloroform extraction and ethanol precipitation, the precipitate is dissolved in 100µl of TE buffer solution. To 8µg DNA/20µl of each sample 8µl of 10 x BclI buffer solution, 4µl of 0.2% bovine serum albumin, 44µl of water and 2 units/4µl of BclI are added and the reaction mixture is incubated at 50°C for 40 min. After phenol chloroform extraction and ethanol precipitation, the precipitate is dissolved in 40µl of TE buffer solution.

### (D) Ligation

To a mixture containing about 90ng/30µl of the DNA sample of 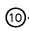 -B, 800 ng/4µl of the DNA sample of -C, 3µl of 10 x ligation buffer, 3µl of 100mM DTT, 3µl of 10mM ATP and 12µl of water 2 units/2µl of T4DNA ligase are added and the reaction mixture is incubated at 16°C for 16hr.

### (E) Transformation

The DNA sample of 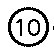 -D is dialyzed against TE buffer solution for 3 hr, and then used to transform the strain C600r⁻m⁻hag::Tn10. On LB medium containing 50µg/ml of ampicillin 2.6 x 10⁵ colonies are formed per 1µg of the treated pFD1 DNA, 3.75 x 10⁵ colonies per 1µg of treated pFD1/HBsAg- I #11 DNA and 2.4 x 10⁵ colonies per 1µg of treated pFD1/HBsAg- II #1 DNA.

### (F) Motility of transformants and Hind III site on plasmids carried thereby

Motility of the transformants of 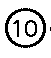 -E is investigated. Eleven of 45 strains transformed by the treated pFD1 show motility. With regard to strains transformed by the treated plasmids pFD1/HBsAg-I #11 and pFD1/HBsAg-II #1, 12 of 45 transformants show motility. DNA samples of these 23(11 + 12) strains are prepared for the rapid plasmid DNA analysis. A mixture containing 7 µl of the DNA sample for the rapid plasmid DNA analysis, 1µl of 10 x EcoRI buffer solution, 1µl of 70mM 2-mercaptoethanol, 0.5µl of 0.2% bovine serum albumin, 4 units/0.5 µl of EcoRI and 5 units/0.5 µl of Hind III is incubated at 37°C for 30 min. DNA fragments generated by 0.8% agarose gel electrophoresis are investigated. A plasmid which shows 3 fragments containing 1.4 kbp and 0.7 kbp fragments is found from the 11 strains transformed by the treated pFD1, and in named pHFD1. The strain having this plasmid is purified to establish the strain C600r⁻m⁻hag::Tn10(pHFD1). A plasmid which shows 2 fragments containing 1.4 kbp fragment is found from the 12 strains transformed by the treated pFD1/HBsAg-I #11 and is named pHFD1/HBsAg-I #11. The strain having this plasmid is purified to establish the strain C600r⁻m⁻hag::Tn10 (pHFD1/HBsAg- I #11). Similarly, a plasmid which shows 2 fragments containing 1.4 kbp fragment in found from the 12 strains transformed by the treated pFD1/HBsAg-II #1 and is named pHFD1/HBsAg- II #1. The strain having the plasmid in purified to establish the strain C600r⁻m⁻hag::Tn10 (pHFD1/HBsAg-II #1).

### (G) Motility depending on plasmid which carries hag gene having pHD1 mutation

The motility of the 5 strains C600r⁻m⁻ (pBR322), C600r⁻m⁻hag::Tn10 (pFD3), C600r⁻m⁻hag::Tn10 (pHFD1), C600r⁻m⁻hag::Tn10 (pHFD1/HBsAg- I I #11) and C600r⁻m⁻hag::Tn10 (pHFD1/HBsAg- II #1) is investigated. The 5 strains are purified in LB broth containing 50 µg/ml of ampicillin. After incubating overnight at 37°C, colonies are inoculated into the medium for the utility test (1% Bactotryptone, 0.25% sodium chloride, 5µg/ml of thiamine and 0.3% agar, pH7.0). After incubating at 37°C or 30°C for 6 hr, the diameter of motile zone is measured (Table 3).

**Table 3**

| Strain | Diameter of motile zone(mm) | |
|---|---|---|
| | 37°C | 30°C |
| C600r⁻m⁻(pBR322) | 43 | 34 |
| C600r⁻m⁻hag::Tn10(pFD3) | 34 | 20 |
| C600r⁻m⁻hag::Tn10(pHFD1) | 41 | 34 |
| C600r⁻m⁻hag::Tn10(pHFD1/HBsAg I #11) | 38 | 32 |
| C600r⁻m⁻hag::Tn10(pHFD1/HBsAg II #1) | 43 | 31 |

The strain C600r⁻m⁻hag::Tn10 having pHFD1, pHFD1/HBsAg- I #11 or pHFD1/HBsAg- II #1 presents motility better than the strain having pFD3 and similar to that of the natural strain C600r⁻m⁻ (pBR322).

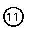 Excretion of flagellin fused with polypeptide HBsAg- III by pFD1, pFD303, pFD306, pFD307, pFD311 and pFD315.

### (A) Digestion of cc-pFD1, cc-pFD303, cc-pFD306, cc-pFD307 , cc-pFD311 and cc-pFD315 with Hind III and repair with klenow fragment

According to ⑧-A, cc-pFD1, cc-PFD303, cc-pFD306, cc-pFD307, cc-pFD311 and cc-pFD315 are digested with Hind III and repaired with klenow fragment.

### (B) Construction of synthesized polynucleotide

Synthesized polynucleotides HBsAgA-1*¹, HBsAgA-2*², HBsAga-3*³, HBsAgA-4*⁴ and HBsAgA-5*⁵ as noted below are synthesized according to the solid phase phosphoroamidite method using ZEON GENET A- II (Zeon). 
*1 5'TTGACACGTATCCTCACAATACCGCAGTC 3' *2 5'ACTAGACTCGTGGGG 3'

*3 5'GAGGATACGTGTCAA 3' *4 5'CTAGTGACTGCGGTATTGT 3'

*5 5'CCCCACGAGT 3'
To HBsAgA-2 (500pM/12.2µl), HBsAgA-3 (500pM/12.2µl) and HBsAgA-4 (500pM/15.4µl) each of which is in a 1.5 ml-eppendorf tube 19.8µl, 19.8µl and 16.6µl of water are added, respectively, To each tube 10µl of of 10 x kinase buffer solution I (0.5M Tris-HCl (pH 7.6), 0.1M magnesium chloride, 50mM dithiothreitol, 1mM spermidine and 1mM EDTA), 1µl of γ⁻³²P ATP (5,000Ci/mM; 10µCL/µl, Amersham), 5µl of 10µM ATP and 2µl of T4 polynucleotide kinase (Takara Shuzo Co., Ltd.) and the reaction mixture is incubated at 37°C for 30 min. Further, 2.5µl of 1mM ATP and 5 units/2µl of T4 polynucleotide kinase are added therto and the reaction mixture is incubated at 37°C for 1hr, then heated to 90°C for 5 min and rapidly chilled in ice.

All of the above reaction mixtures of HBsAgA-2, HBsAgA-3 and HBsAgA-4 are mixed with HBsAgA-1 (500pM/23.5µl) and HBsAgA-5 (500pM/8.1µl). The mixture is put in a dialyze membrane (spectra pore/ molecular weight cut off 1,000, spectrum medical industry) and dialysed overnight at 4°C against water. The resultant is placed in a 1.5 ml-eppendorf tube and concentrated with a centrifuge evaporator. The concentrated mixture is dissolved in 15µl of Tris-HCl containing 10mM magnesium chloride, then heated to 60°C for 30 min and gradually cooled to room temperature in 6 hr. To the resulting mixture 16µl of 5 x ligation buffer, 4µl of 200 mM DTT, 10mM ATP, 22.4 units/8µl of T4DNA ligase and 37µl of water are added and the reaction mixture is incubated at 15°C for 16hr. The resultant is applied to 15% polyacrylamide gel electrophoresis and then to autoradiography. The band corresponding to 44bp is cut off, placed in a 1.5ml-eppendorf tube and broken with a glass rod. The resultant is extracted with 1 ml of water at 4°C for 16 hr. After centrifugation, the superpatant ( about 700µl) is concentrated with a centrifuge evaporator. The concentrate (about 200µl) is centrifuged again to remove residues of the gel.

### (C) Ligation of synthesized DNA with pFD1, pFD303, pFD306, pFD307, pFD311 and pFD315.

A mixture containing 500ng/1µl of the DNA sample of 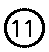 -A, 250pM/100µl of the synthesized DNA of 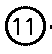 -B, 4µl of 5 x ligation buffer, 2µl of 200mM DTT, 2µl of 10mM ATP, 2µl of T4DNA ligase and 4µl of water is incubated at 15°C for 16hr. After dialysing against TE buffer solution, the whole volume of the resultant is used for transformation.

### D) Selection and motility of transformants

A strain C600r⁻m⁻hag::Tn10 is transformed with the DNA sample of 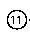 -C. On LB medium containing 50µg/ml of ampicillin 5x10⁴ colonies are formed per 1µg pFD1DNA, 2x10⁵ colonies/1µg pFD303DNA, 1.8x10⁵colonies/1µg pFD306DNA, 2.3x10⁴colonies/1µg pFD307DNA, 1.3x10⁵colonies/1µg pFD311DNA and 8x10⁴colonies/1µg pFD315DNA.

Motility of 100 strains of each group is investigated on the medium for the motility test. From colonies showing motility, 6 motile strains of each group are incubated overnight in LB medium containing 50µg/ml of ampicillin. Then it is investigated whether a Hind III restriction site lies on the plasmid carried by the strains. Since a Hind III site on the plasmid is cut with Hind III and repaired with klenow fragment, Hind III is generated again when the synthesized DNA HBsAg- II is inserted thereto in a right direction and, as a result, provides T of 5' terminal of the synthesized DNA to the restriction site. When the synthesized DNA is inserted in a reverse direction or not inserted, Hind III site is not generated. Accordingly, the synthesized DNA is inserted in a right direction if a short fragment is generated when the plasmid is digested with Hind III and Sst II (Sst II site lies downstream of the insertion site at a short distance), and the synthesized DNA is inserted in a reverse direction or not inserted if a short fragment is not generated. Thus, it can be investigated whether the synthesized DNA is inserted in a right direction. Therefore, a plasmid DNA prepared from each strain by the rapid method is digested with Hind III and Sst II. A mixture containing 8µl of the sample of a plasmid, 2µl of 10 x Sst II buffer solution (500mM Tris-HCl (pH 8.0), 100mM magnesium chloride and 500mM sodium chloride), 2µl of 70mM 2-mercaptoethanol, 10 units/1µl of Hind III, 7 units/1µl of Sst II and 6µl of water is incubated for digestion. After 5% polyacrylamide electrophoresis (150V, 95 min) and ethidium bromide-staining, it is investigated whether a band corresponding to the short fragment is observed. As a result, 2 strains are established from each group. They show motility and have a plasmid into which the synthesized DNA is inserted in a right direction. These strains excrete flagellin fused with the peptide LeuThrArgIleLeuThrIleProGlnSerLeuAsp-SerTrpGly encoded by the synthesized DNA (HBsAg- III) inserted in the plasmid. Also, this fused flagellin can construct the flagella.

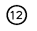 Excretion of flagellin fused with polypeptide HBsAg- IV by pFD1, pFD303, pFD306, pFD307, pFD311 and pFD315

### (A) Digestion of cc-pFD1, cc-pFD303, cc-pFD306, cc-PFD307, cc-pFD311 and cc-pFD315 with Hind III and repair with klenow fragment

According to 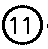 -A, cc-pFD1, cc-pFD303, cc-pFD306, cc-pFD307, cc-pFD311 and cc-pFD315 are digested with Hind III and repaired with klenow fragment.

### (B) Construction of synthesized polynucleotide

Synthesized polynucleotides HBsAgB-1*¹, HBsAgB-2*², HBsAgB-3*³, HBsAgB-4*⁴, HBsAgB-5*⁵ and HBsAgB-6*⁶ as noted below are synthesized according to 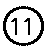 -B. 
*1 5'TTGGTTCTTCTGGATTAT 3' *2 5'CAGAAGAACCAA 3'

*3 5'CAAGGTATGTTGCCCGT 3' *4 5'AACATACCTTGATAATC 3'

*5 5'TTGTCCTCTAGG 3' *6 5'CCTAGAGGACAAACGGGC 3'
To HBsAgB-2 (500pM/9.7µl), HBsAgB-5(500mM/9.7µl), HBsAgB-3 (500pM/13.8µl) and HBsAgB-4 (500pM/13.8µl) each of which is in a 1.5ml-eppendorf tube 22.3µl, 22.3µl, 18.2µl and 18.2µl of water are added, respectively. According to 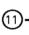 -B, the synthesized polynucleotides are phosphatized at 5' terminal. The above whole reaction mixtures are mixed with 14.6µl of HBsAgB-1 and 14.6µl of HBsAgB-6 and ligated according to 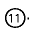 -B. Then, 200µl of the resultant are separated by polyacrylamide gel electrophoresis.

### (C) Ligation of synthesized DNA with pFD1, pFD303, pFD306, pFD307, pFD311 and pFD315

According to 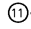 -C, HBsAg- IV is ligated with a plasmid and the whole volume of the resultant is used for transformation.

### (D) Selection of transformants and their motility

A strain C600r⁻m⁻hag::Tn10 is transformed with the sample DNA of 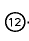 -C. On LB media containing 50µg/ml of ampicillin
4.4x10⁴ colonies/1µg pFD1 DNA, 2.9x10⁴ colonies/1µg pFD303 DNA, 3.8x10⁴colonies/1µg pFD306 DNA, 3.2x10⁴colonies/1 µg pFD307 DNA, 1.4x10⁵colonies/1µl pFD311 DNA and 5.8x10⁴ colonies/1µg pFD315 DNA are formed. From each group 6 strains showing motility are selected, and then investigated whether they have a plasmid into which the synthesized DNA HBsAg-IV is inserted in a right direction. Two strains are established from each group. These strains excrete flagellin fused with the peptide LeuValLeuLeuAspTyr-GlnGlyMetLeuProValCysProLeuGly and form flagella. The said peptide is encoded by the synthesized DNA (HBsAg-IV) which is inserted into the plasmid.

### Referential Examination

### Transformation of E. coli JA11 with pBR322/hag93

*E. coli* JA11 (FERM P-8853) is incubated to be competent according to the method of Hanahan et al. (J. Mol. Biol 166, 557-580, 1983). The competent cells (210µl) of strain JA11 frozen at -70°C are melted and 200ng of pBR322/hag93 cc-DNA dissolved in TE buffer solution are added in ice at 0°C. After incubation in ice at 0°C for 30 min and heating to 42°C for 100 sec, 0.8ml of SOC broth are added to the mixture. After incubation at 37°C for 1hr with shaking, the culture is diluted 10⁴ times, and 0.1 ml of this suspension are spread on LB agar medium containing 50µg/ml of ampicillin. After overnight incubation at 37°C, 52 colonies are formed. This means that the strain JA11 is transformed with pBR322/hag93 at a ratio of 2.6x10⁷ colonies/1 µg cc-DNA.

## Claims (Claims for the following Contracting State(s): BE, CH, DE, FR, GB, GR, IT, LI, LU, NL, SE)

1. A DNA having a DNA sequence containing the hag gene which encodes a flagellin protein, wherein said flagellin is obtained by deleting bps 583 to 1143 of said DNA sequence.

2. The DNA according to claim 1, wherein said DNA sequence contains from bps -28 to -21 of said hag gene, wherein said bps -28 to -21 are the nucleotide bases 5'CAAGCTTG designated as the pHD1 mutation.

3. The DNA according to claim 1 or 2, wherein said bps 583 to 1143 are responsible for the antigenicity of flagellin.

4. The DNA according to any one of claims 1 to 3, which further contains one or more DNA linkers having suitable restriction sites for insertion of a DNA coding for a foreign peptide.

5. The DNA according to claim 4, wherein said linker is inserted into the resulting space where bps 583 to 1143 of said DNA sequence were deleted.

6. The DNA according to any one of claims 1 to 5, wherein said DNA coding for a foreign peptide is inserted into said linker.

7. The DNA according to any one of claims 1 to 6, wherein said DNA sequence encodes flagellin of Escherichia coli.

8. The DNA according to claim 7, wherein said Escherichia coli is E. coli K-12.

9. The DNA sequence according to claim 8, wherein said E. coli K-12 is E. coli K-12 HK552.

10. A vector having a DNA sequence according to any one of claims 1 to 9, wherein said vector is capable of expressing said flagellin protein and said foreign protein.

11. The vector according to claim 10, wherein upon expression said foreign protein is excreted as a peptide fused with said flagellin protein.

12. The vector according to claims 10 or 11, which is capable of transforming a bacterium which does not form flagella into a bacterium having flagella.

13. A host organism which is transformed with a vector according to any one of claims 10 to 12.

14. A host organism which is transformed with a vector according to any one of claims 10 to 12 which is capable of excreting said foreign protein.

15. The host organism according to claims 13 or 14, wherein said foreign protein is HBsAg.

16. A method of expressing a foreign protein by culturing said host organism according to any one of claims 13 to 15 under conditions sufficient to allow said foreign protein to be expressed and excreted.

## Claims (Claims for the following Contracting State(s): AT, ES)

1. A process for the preparation of a DNA having a DNA sequence containing the hag gene which encodes a flagellin protein, which comprises deleting bps 583 to 1143 of said DNA sequence.

2. The process according to claim 1, wherein said DNA sequence contains from bps -28 to -21 of said hag gene, wherein said bps -28 to -21 are the nucleotide bases 5'CAAGCTTG designated as the pHD1 mutation.

3. The process according to claim 1 or 2, whereby said bps 583 to 1143 are responsible for the antigenicity of flagellin.

4. The process according to any one of claims 1 to 3, which further comprises introducing one or more DNA linkers having suitable restriction sites for insertion of a DNA coding for a foreign peptide.

5. The process according to claim 4, wherein said linker is inserted into the resulting space where bps 583 to 1143 of said DNA sequence were deleted.

6. The process according to any one of claims 1 to 5, wherein said DNA coding for a foreign peptide is inserted into said linker.

7. The process according to any one of claims 1 to 6, wherein said DNA sequence encodes flagellin of *Escherichia coli*.

8. The process according to claim 7, wherein said *Escherichia coli* is *E. coli* K-12.

9. The process according to claim 8, wherein said *E. coli* K-12 is *E. coli* K-12 HK552.

10. A vector having a DNA sequence obtained according to any one of claims 1 to 9, wherein said vector is capable of expressing said flagellin protein and said foreign protein.

11. The vector according to claim 10, wherein upon expression said foreign protein is excreted as a peptide fused with said flagellin protein.

12. The vector according to claims 10 or 11, which is capable of transforming a bacterium which does not form flagella into a bacterium having flagella.

13. A host organism which is transformed with a vector according to any one of claims 10 to 12.

14. A host organism which is transformed with a vector according to any one of claims 10 to 12 which is capable of excreting said foreign protein.

15. The host organism according to claims 13 or 14, wherein said foreign protein is HBsAg.

16. A method of expressing a foreign protein by culturing said host organism according to any one of claims 13 to 15 under conditions sufficient to allow said foreign protein to be expressed and excreted.

## Patentansprüche (Patentansprüche für folgende(n) Vertragsstaat(en): BE, CH, DE, FR, GB, GR, IT, LI, LU, NL, SE)

1. DNA mit einer DNA-Sequenz, die das ein Flagellinprotein codierende Hag-Gen enthält, wobei das Flagellin durch Deletion der Basenpaare 538 bis 1143 der DNA-Sequenz erhalten wird.

2. DNA nach Anspruch 1, wobei die DNA-Sequenz die Basenpaare -28 bis -21 des Hag-Gens enthält, wobei die Basenpaare -28 bis -21 die Nucleotidbasen 5'CAAGCTTG mit der Bezeichnung pHD1-Mutation sind.

3. DNA nach Anspruch 1 oder 2, wobei die Basenpaare 583 bis 1143 für die Antigenität des Flagellins verantwortlich sind.

4. DNA nach einem der Ansprüche 1 bis 3, welche weiter einen oder mehrere DNA-Linker mit geeigneten Restriktionsspaltstellen zur Insertion einer DNA, die ein Fremdpeptid codiert, enthält.

5. DNA nach Anspruch 4, wobei der Linker in den Bereich eingebaut ist, der erhalten wird, wenn die Basenpaare 583 bis 1143 deletiert werden.

6. DNA nach einem der Ansprüche 1 bis 5, wobei die ein Fremdpeptid codierende DNA in den Linker eingebaut ist.

7. DNA nach einem der Ansprüche 1 bis 6, wobei die DNA-Sequenz Flagellin von Escherichia coli codiert.

8. DNA nach Anspruch 7, wobei Escherichia coli E. coli K-12 ist.

9. DNA-Sequenz nach Anspruch 8, wobei E. coli K-12 E. coli K-12 HK552 ist.

10. Vektor mit einer DNA-Sequenz nach einem der Ansprüche 1 bis 9, wobei der Vektor die Fähigkeit besitzt, das Flagellinprotein und das Fremdprotein zu exprimieren.

11. Vektor nach Anspruch 10, wobei nach der Expression das Fremdprotein als Fusionspeptid mit dem Flagellinprotein ausgeschieden wird.

12. Vektor nach Anspruch 10 oder 11, mit der Fähigkeit zur Transformation eines Bakteriums, das keine Flagellen bildet, in ein Bakterium mit Flagellen.

13. Wirtsorganismus, der mit einem Vektor nach einem der Ansprüche 10 bis 12 transformiert ist.

14. Wirtsorganismus, der mit einem Vektor nach einem der Ansprüche 10 bis 12 transformiert ist, mit der Fähigkeit zur Ausscheidung des Fremdproteins.

15. Wirtsorganismus nach den Ansprüchen 13 oder 14, wobei das Fremdprotein HBsAg ist.

16. Verfahren zur Expression eines Fremdproteins durch Züchten des Wirtsorganismus nach einem der Ansprüche 13 bis 15 unter Bedingungen, die die Expression und Ausscheidung des Fremdproteins ermöglichen.

## Patentansprüche (Patentansprüche für folgende(n) Vertragsstaat(en): AT, ES)

1. Verfahren zur Herstellung einer DNA mit einer DNA-Sequenz, die das ein Flagellinprotein codierende Hag-Gen enthält, umfassend die Deletion der Basenpaare 538 bis 1143 der DNA-Sequenz.

2. Verfahren nach Anspruch 1, wobei die DNA-Sequenz die Basenpaare -28 bis -21 des Hag-Gens enthält, wobei die Basenpaare -28 bis -21 die Nucleotidbasen 5'CAAGCTTG mit der Bezeichnung pHD1-Mutation sind.

3. Verfahren nach Anspruch 1 oder 2, wobei die Basenpaare 583 bis 1143 für die Antigenität des Flagellins verantwortlich sind.

4. Verfahren nach einem der Ansprüche 1 bis 3, weiterhin umfassend die Einführung eines oder mehrerer DNA-Linker mit geeigneten Restriktionsspaltstellen zur Insertion einer DNA, die ein Fremdpeptid codiert.

5. Verfahren nach Anspruch 4, wobei der Linker in den Bereich eingebaut ist, der erhalten wird, wenn die Basenpaare 583 bis 1143 deletiert werden.

6. Verfahren nach einem der Ansprüche 1 bis 5, wobei die ein Fremdpeptid codierende DNA in den Linker eingebaut ist.

7. Verfahren nach einem der Ansprüche 1 bis 6, wobei die DNA-Sequenz Flagellin von Escherichia coli codiert.

8. Verfahren nach Anspruch 7, wobei Escherichia coli E. coli K-12 ist.

9. Verfahren nach Anspruch 8, wobei E. coli K-12 E. coli K-12 HK552 ist.

10. Vektor mit einer DNA-Sequenz, die nach einem der Ansprüche 1 bis 9 erhalten wurde, wobei der Vektor die Fähigkeit besitzt, das Flagellinprotein und das Fremdprotein zu exprimieren.

11. Vektor nach Anspruch 10, wobei nach der Expression das Fremdprotein als Fusionspeptid mit dem Flagellinprotein ausgeschieden wird.

12. Vektor nach Anspruch 10 oder 11, mit der Fähigkeit zur Transformation eines Bakteriums, das keine Flagellen bildet, in ein Bakterium mit Flagellen.

13. Wirtsorganlsmus, der mit einem Vektor nach einem der Ansprüche 10 bis 12 transformiert ist.

14. Wirtsorganismus, der mit einem Vektor nach einem der Ansprüche 10 bis 12 transformiert ist, mit der Fähigkeit zur Ausscheidung des Fremdproteins.

15. Wirtsorganismus nach den Ansprüchen 13 oder 14, wobei das Fremdprotein HBsAg ist.

16. Verfahren zur Expression eines Fremdproteins durch Züchten des Wirtsorganismus nach einem der Ansprüche 13 bis 15 unter Bedingungen, die die Expression und Ausscheidung des Fremdproteins ermöglichen.

## Revendications (Revendications pour l'(les) Etat(s) contractant(s) suivant(s): BE, CH, DE, FR, GB, GR, IT, LI, LU, NL, SE)

1. ADN comportant une séquence d'ADN contenant le gène hag qui code pour une protéine constituée de flagelline, dans lequel ladite flagelline est obtenue par délétion des bps 583 à 1143 de ladite séquence d'ADN.

2. ADN suivant la revendication 1, caractérisé en ce que la séquence d'ADN contient les bps de -28 à -21 du gène hag, ces bps -28 à -21 étant les bases nucléotidiques 5'CAAGCTTG appelées mutation pHD1.

3. ADN suivant l'une ou l'autre des revendications 1 et 2, caractérisé en ce que les bps 583 à 1143 sont responsables de l'antigénicité de la flagelline.

4. ADN suivant l'une quelconque des revendications 1 à 3, qui contient de plus un ou plusieurs adaptateurs d'ADN comportant des sites de restriction appropriés pour l'insertion d'un ADN codant pour un peptide étranger.

5. ADN suivant la revendication 4, caractérisé en ce que l'adaptateur est inséré dans l'espace résultant où les bps 583 à 1143 de la séquence d'ADN ont été délétées.

6. ADN suivant l'une quelconque des revendications 1 à 5, caractérisé en ce que l'ADN codant pour un peptide étranger est inséré dans l'adaptateur précité.

7. ADN suivant l'une quelconque des revendications 1 à 6, caractérisé en ce que la séquence d'ADN code pour de la flagelline d'Escherichia coli.

8. ADN suivant la revendication 7, caractérisé en ce que l'Escherichia coli est du E. coli K-12.

9. Séquence d'ADN suivant la revendication 8, caractérisée en ce que l'E. coli K-12 est de l'E. coli K-12 HK552.

10. Vecteur comportant une séquence d'ADN suivant l'une quelconque des revendication 1 à 9, caractérisé en ce que ledit vecteur peut exprimer la protéine constituée de flagelline et la protéine étrangère.

11. Vecteur suivant la revendication 10, caractérisé en ce que lors de l'expression la protéine étrangère est excrétée sous la forme d'un peptide fusionné avec la protéine constituée de flagelline.

12. Vecteur suivant l'une ou l'autre des revendications 10 et 11, qui peut transformer une bactérie qui ne forme pas de flagelles en une bactérie comportant des flagelles.

13. Organisme hôte qui est transformé avec un vecteur suivant l'une quelconque des revendications 10 à 12.

14. Organisme hôte qui est transformé avec un vecteur suivant l'une quelconque des revendications 10 à 12 qui peut excréter la protéine étrangère.

15. Organisme hôte suivant l'une ou l'autre des revendications 13 et 14, caractérisé en ce que la protéine étrangère est de l'HBsAg.

16. Procédé d'expression d'une protéine étrangère par la culture de l'organisme hôte suivant l'une quelconque des revendications 13 à 15, sous des conditions suffisantes pour permettre à ladite protéine étrangère d'être exprimée et excrétée.

## Revendications (Revendications pour l'(les) Etat(s) contractant(s) suivant(s): AT, ES)

1. Procédé de préparation d'un ADN comportant une séquence d'ADN contenant le gène hag qui code pour une protéine constituée de flagelline, comprenant une délétion des bps 583 à 1143 de ladite séquence d'ADN.

2. Procédé suivant la revendication 1, caractérisé en ce que la séquence d'ADN contient les bps de -28 à -21 du gène hag, ces bps -28 à -21 étant les bases nucléotidiques 5'CAAGCTTG appelées mutation pHD1.

3. Procédé suivant l'une ou l'autre des revendications 1 et 2, caractérisé en ce que les bps 583 à 1143 sont responsables de l'antigénicité de la flagelline.

4. Procédé suivant l'une quelconque des revendications 1 à 3, qui comprend de plus une introduction d'un ou de plusieurs adaptateurs d'ADN comportant des sites de restriction appropriés pour l'insertion d'un ADN codant pour un peptide étranger.

5. Procédé suivant la revendication 4, caractérisé en ce que l'adaptateur est inséré dans l'espace résultant où les bps 583 à 1143 de la séquence d'ADN ont été délétées.

6. Procédé suivant l'une quelconque des revendications 1 à 5, caractérisé en ce que l'ADN codant pour un peptide étranger est inséré dans l'adaptateur précité.

7. Procédé suivant l'une quelconque des revendications 1 à 6, caractérisé en ce que la séquence d'ADN code pour de la flagelline d'Escherichia coli.

8. Procédé suivant la revendication 7, caractérisé en ce que l'Escherichia coli est du E. coli K-12.

9. Procédé suivant la revendication 8, caractérisé en ce que l'E. coli K-12 est de l'E. coli K-12 HK552.

10. Vecteur comportant une séquence d'ADN obtenue suivant l'une quelconque des revendication 1 à 9, caractérisé en ce que ledit vecteur peut exprimer la protéine constituée de flagelline et la protéine étrangère.

11. Vecteur suivant la revendication 10, caractérisé en ce que, lors de l'expression, la protéine étrangère est excrétée sous la forme d'un peptide fusionné avec la protéine constituée de flagelline.

12. Vecteur suivant l'une ou l'autre des revendications 10 et 11, qui peut transformer une bactérie qui ne forme pas de flagelles en une bactérie comportant des flagelles.

13. Organisme hôte qui est transformé avec un vecteur suivant l'une quelconque des revendications 10 à 12.

14. Organisme hôte qui est transformé avec un vecteur suivant l'une quelconque des revendications 10 à 12 qui peut excréter la protéine étrangère.

15. Organisme hôte suivant l'une ou l'autre des revendications 13 et 14, caractérisé en ce que la protéine étrangère est de l'HBsAg.

16. Procédé d'expression d'une protéine étrangère par la culture de l'organisme hôte suivant l'une quelconque des revendications 13 à 15, sous des conditions suffisantes pour permettre à ladite protéine étrangère d'être exprimée et excrétée.
